(19) Europäisches Patentamt
European Patent Office
Office européen des brevets

(11) **EP 4 151 709 A1**

(12) **EUROPEAN PATENT APPLICATION**
published in accordance with Art. 153(4) EPC

(43) Date of publication:
**22.03.2023 Bulletin 2023/12**

(21) Application number: **20934894.5**

(22) Date of filing: **12.05.2020**

(51) International Patent Classification (IPC):
*C12M 1/00* (2006.01)          *G06T 7/55* (2017.01)
*C12Q 1/6874* (2018.01)       *G16B 40/20* (2019.01)

(52) Cooperative Patent Classification (CPC):
**C12M 1/00; C12Q 1/6874; G06T 7/55; G16B 40/20**

(86) International application number:
**PCT/JP2020/018902**

(87) International publication number:
**WO 2021/229668 (18.11.2021 Gazette 2021/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **HITACHI HIGH-TECH CORPORATION**
**Tokyo 105-6409 (JP)**

(72) Inventor: **YOKOYAMA Toru**
**Tokyo 100-8280 (JP)**

(74) Representative: **MERH-IP Matias Erny Reichl
Hoffmann
Patentanwälte PartG mbB
Paul-Heyse-Strasse 29
80336 München (DE)**

(54) **NUCLEIC ACID ANALYSIS DEVICE, NUCLEIC ACID ANALYSIS METHOD, AND MACHINE LEARNING METHOD**

(57)  An object of the invention is to provide a nucleic acid analysis technique robust to the registration accuracy of images.

According to a preferred aspect of the invention, there is provided a nucleic acid analyzer including: a base prediction unit configured to perform base prediction using, as an input, a plurality of images obtained by detecting luminescence from a biologically related substance disposed on a substrate; a registration unit configured to perform registration of the plurality of images relative to a reference image; and an extraction unit configured to extract a spot from the plurality of images, in which the base prediction unit receives, as an input, an image including peripheral pixels around a position of the spot extracted from the plurality of images, extracts feature data of the image, and predicts a base based on the feature data.

[FIG. 14]

**Description**

Technical Field

The present invention relates to a nucleic acid analysis technique for measuring a biologically related substance.

Background Art

In recent years, a method has been proposed in which a large number of DNA fragments to be analyzed are supported by a flow cell formed of a glass substrate, a silicon substrate, or the like, and a base sequence of the large number of DNA fragments are determined in parallel in a nucleic acid analyzer. In the method, a substrate with a fluorescent dye corresponding to a base is introduced into an analyzing area on a flow cell containing the large number of DNA fragments, the flow cell is irradiated with excitation light, fluorescence emitted from each DNA fragment is detected, and the base is identified (called).

In order to analyze a large amount of DNA fragments, the analyzing area is usually divided into a plurality of fields of view, and analysis is performed in all the fields of view by changing a field of view every time irradiation is performed. Then, a new substrate with a fluorescent dye is introduced using a polymerase extension reaction, and each detection field of view is analyzed by the same operation as described above. By repeating the cycle, the base sequence can be efficiently determined (see PTL 1).

In the analysis as described above, fluorescence emitted from an amplified DNA sample (hereinafter referred to as a "colony") immobilized on a substrate is imaged, and bases are specified by image processing. That is, each colony in a fluorescent image is identified, a fluorescence intensity corresponding to bases at a position of each colony is acquired, and the bases are identified based on the fluorescence intensity (see PTL 2).

In general, even in fluorescence imaging performed in the same field of view, an imaged position varies on the flow cell due to the limit of control accuracy of a driving device for changing a field of view. Therefore, a certain colony is imaged at different coordinate positions in each fluorescent image. Therefore, in order to accurately identify each colony, it is necessary to accurately determine a coordinate position of each colony on a flow chip.

For this purpose, there are a method of placing, on a substrate, a reference marker for determining a position on a substrate and a method of detecting a position of each colony of a captured image by image correlation matching with a reference image. The reference image herein is an image generated based on design data of a flow chip, in which position data that is position coordinates of a colony on the image has been known. Alternatively, any one of a plurality of images captured in each field of view may be used as a reference image, and a colony on another image may be associated with the colony on the reference image. Hereinafter, processing of associating position coordinates between a reference image and a target image is referred to as registration.

Citation List

Patent Literature

PTL 1: JP-A-2020-60
PTL 2: WO 2017-203679

Summary of Invention

Technical Problem

However, the registration depends on an image pattern of a colony and the degree of focus of an image. In general, as the cycle of the extension reaction or the imaging is repeated, the DNA is degraded and the fluorescence intensity is attenuated. Therefore, as the number of cycles increases, the registration accuracy decreases. In addition, the registration accuracy also decreases when the focus during imaging is poor. As will be described below, when registration is performed using fluorescent images of different cameras, there is a possibility that registration accuracy decreases due to a large difference in lens distortion. When the registration accuracy decreases, the reliability of the fluorescence intensity acquired at the position of each colony is lowered, and therefore, there is a high possibility that an erroneous base is called.

The invention has been made in view of such circumstances, and an object thereof is to provide a nucleic acid analysis technique robust to the registration accuracy of images.

Solution to Problem

According to a preferred aspect of the invention, there is provided a nucleic acid analyzer including: a base prediction unit configured to perform base prediction using, as an input, a plurality of images obtained by detecting luminescence from a biologically related substance disposed on a substrate; a registration unit configured to perform registration of the plurality of images relative to a reference image; and an extraction unit configured to extract a spot from the plurality of images, in which the base prediction unit receives, as an input, an image including peripheral pixels around a position of the spot extracted from the plurality of images, extracts feature data of the image, and predicts a base based on the feature data.

In a more specific example of the apparatus, the plurality of images are obtained by detecting, by a sensor, a plurality of types of luminescence from a plurality of types of fluorescent substances incorporated into the biologically related substance, and the plurality of types of luminescence are different in at least one of the sensor for detection and an optical path to the sensor for detection.

In another more specific example of the apparatus, the base prediction unit is implemented by a predictor capable of performing supervised learning.

In another more specific example of the apparatus, the base prediction unit receives, in addition to an image in a cycle to be predicted, an image in at least one cycle selected from a previous cycle and a next cycle as an input.

According to another preferred aspect of the invention, there is provided a nucleic acid analysis method for performing base prediction by a base predictor receiving, as an input, a plurality of images obtained by detecting luminescence from a biologically related substance, and the method includes executing a colony position determining stage and a base sequence determining stage. In the colony position determining stage, registration processing of registering the plurality of images, and colony position determining processing of determining a colony position of the biologically related substance by extracting a spot from the plurality of images are executed. In the base sequence determining stage, the base predictor receives, as an input, an image including peripheral pixels around the colony position extracted from the plurality of images, extracts feature data of the image, and predicts a base based on the feature data.

According to still another preferred aspect of the invention, there is provided a machine learning method of a base predictor for performing base prediction using, as an input, a plurality of images obtained by detecting luminescence from a biologically related substance. The method includes: a first base prediction step of generating a first base prediction result based on the plurality of images; a first training data generation step of generating first training data based on an alignment result between the first base prediction result and a reference sequence; a predictor updating step of updating a parameter of the base predictor using the first training data generated in the first training data generation step; a second base prediction step of generating a second base prediction result based on the plurality of images by using the base predictor updated in the predictor updating step; a second training data generation step of generating second training data based on an alignment result between the second base prediction result and the reference sequence; and a training data updating step of updating the first training data using the second training data.

Advantageous Effects of Invention

The invention can provide a nucleic acid analysis technique robust to the registration accuracy of images.

Brief Description of Drawings

FIG. 1 is a block diagram showing a schematic configuration example of a nucleic acid analyzer according to embodiments.
FIG. 2 is a chart showing processing steps for decoding a DNA base sequence according to the embodiments.
FIG. 3 is a plan view illustrating a concept of a field of view on a flow cell according to the embodiments.
FIG. 4 is an illustration diagram showing a concept of spots of four types of fluorescent images in each field of view according to the embodiments.
FIG. 5 is an illustration diagram showing a concept of determination of base sequences according to the embodiments.
FIG. 6 is a block diagram illustrating an example of a configuration of a computer in the nucleic acid analyzer according to a first embodiment.
FIG. 7 is a flowchart showing a flow of a base calling stage according to the first embodiment.
FIG. 8 is a flowchart showing a flow of a colony position determining stage according to the first embodiment.
FIG. 9 is an illustration diagram showing a concept of a positional deviation between cycles according to the embodiments.
FIG. 10 is an illustration diagram showing a concept of measuring a positional deviation amount at a plurality of positions in an image according to the embodiments.

FIG. 11 is an illustration diagram showing a concept of a colony extraction with a plurality of cycles according to the first embodiment.

FIG. 12 is an illustration diagram showing a concept of determination of a colony position with a plurality of cycles according to the first embodiment.

FIG. 13 is a flowchart showing a flow of a base sequence determining stage according to the first embodiment.

FIG. 14 is an illustration diagram showing a concept of an ROI image for colonies according to the first embodiment.

FIG. 15 is a block diagram showing a concept of a base predictor according to the first embodiment.

FIG. 16 is a block diagram showing an example of a configuration of a Convolutional Neural Network according to the first embodiment.

FIG. 17 is an illustration diagram showing a concept of a base predictor having ROI images for a plurality of cycles as inputs according to a second embodiment.

FIG. 18 is a block diagram showing a concept of base prediction using a plurality of base predictors according to a third embodiment.

FIG. 19 is a flowchart showing a flow of training of base predictor parameters according to a fourth embodiment.

FIG. 20 is an illustration diagram illustrating a concept of alignment processing according to the fourth embodiment.

FIG. 21 is an illustration diagram illustrating a relation between bases of all colonies and bases of an aligned colony according to the fourth embodiment.

FIG. 22 is an illustration diagram illustrating a concept of correct information constituting training data according to the fourth embodiment.

FIG. 23 is an illustration diagram illustrating a concept of updating the training data according to the fourth embodiment.

FIG. 24 is an illustration diagram illustrating a concept of improving prediction performance by repetition of the training according to the fourth embodiment.

FIG. 25 shows graphs illustrating a concept of improving an alignment rate and an error rate by repeating the training according to the fourth embodiment.

FIG. 26 is an illustration diagram showing a concept of enriching training data by blur processing according to a sixth embodiment.

FIG. 27 is an illustration diagram showing a concept of enriching the training data by shift processing according to the sixth embodiment.

FIG. 28 is an illustration diagram showing a concept of screening training data based on a signal intensity of a colony position according to a seventh embodiment.

FIG. 29 is an illustration diagram showing a concept of screening the training data based on reliability of a colony position according to the seventh embodiment.

FIG. 30A is an illustration diagram illustrating a system configuration in which nucleic acid analyzers, base prediction units, and learning units according to an eighth embodiment are different.

FIG. 30B is an illustration diagram illustrating a system configuration in which nucleic acid analyzers, base prediction units, and learning units according to the eighth embodiment are different.

FIG. 30C is an illustration diagram illustrating a system configuration in which nucleic acid analyzers, base prediction units, and learning units according to the eighth embodiment are different.

FIG. 31 is an image diagram showing an example of a screen for selecting a plurality of base predictors according to a ninth embodiment.

FIG. 32 is an image diagram showing an example of a setting screen when base predictors are selectively used for each cycle according to the ninth embodiment.

FIG. 33 is an image diagram showing an example of a setting screen for adding or deleting a data set used for training of a base predictor according to the ninth embodiment.

Description of Embodiments

Hereinafter, embodiments of the invention will be described with reference to the accompanying drawings. In the accompanying drawings, elements with the same functions may be denoted by the same number. The accompanying drawings show specific embodiments and implementation examples in accordance with the principle of the invention, but these are for understanding the invention and are not used to limit the invention. That is, it should be understood that the description of the present specification is merely a typical example and does not limit the scope of the claims or the application examples in any sense.

The various embodiments described below are described in a sufficiently detailed manner in order for those skilled in the art to implement the invention, but it should be understood that other implementations and forms are possible, and changes in configurations and structures and replacement of various elements are possible without departing from the scope and spirit of the technical idea of the invention. Therefore, the following description should not be construed as being limited to the embodiments. The nucleic acid analyzers according to the various embodiments are intended to measure and analyze DNA fragments, and RNA, a protein, or the like in addition to DNA may be used as a target, and the invention can be applied to all biologically related substance.

As will be described below, the embodiments of the invention may be implemented by software running on a general-purpose computer, and may be implemented by dedicated hardware or a combination of software and hardware.

Hereinafter, each processing in the embodiments of the present disclosure will be described using, as a subject (operation subject), each processing unit (for example, a registration unit, a colony extraction unit, a base prediction unit, and a learning unit) as a "program", and the program is executed by the processor to perform determined processing while using a memory and a communication port (communication control device), so that the description may be made with the processor as the subject. Some or all of the programs may be implemented by the dedicated hardware or may be modularized.

Hereinafter, various embodiments of the invention will be sequentially described with reference to the drawings. A representative embodiment provides a base calling method in which registration between fluorescent images and detection of a colony in each fluorescent image is performed, and a region of interest (ROI) image around a colony in a fluorescent image is received as an input. The representative embodiment also provides a method of training a base predictor by repeating training data update and base calling based on a comparison between a called base sequence and a reference sequence.

[First Embodiment]

(1) Nucleic acid analyzer

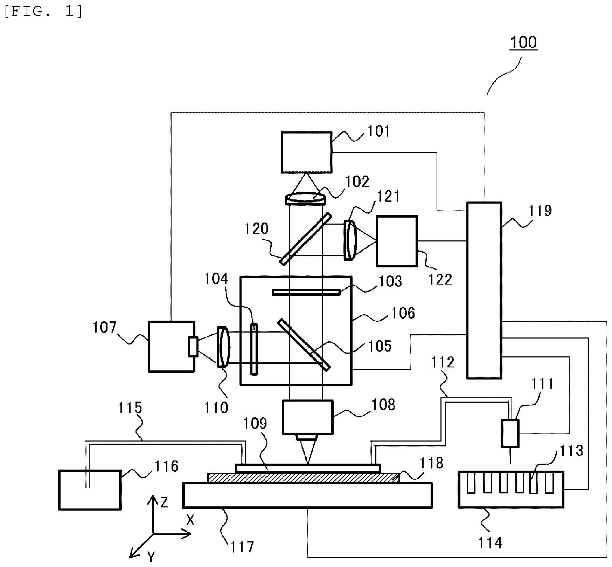

FIG. 1 shows a schematic configuration example of a nucleic acid analyzer according to embodiments. A nucleic acid analyzer 100 includes a flow cell 109, a fluid delivery unit, a conveyance unit, a temperature control unit, an optical unit, and a computer 119. The flow cell 109 is provided with a substrate for nucleic acid analysis of the embodiments described below.

The fluid delivery unit provides units for supplying a reagent to the flow cell 109. The fluid delivery unit includes, as the units, a reagent storage unit 114 for accommodating a plurality of reagent containers 113, a nozzle 111 for accessing the reagent containers 113, a pipe 112 for introducing a reagent into the flow cell 109, a waste liquid tank 116 for disposal of a waste liquid such as a reagent that has reacted with a DNA fragment, and a pipe 115 for introducing the waste liquid into the waste liquid tank 116.

The conveyance unit moves an analyzing area 123 of the flow cell 109 to be described below to a predetermined position. The conveyance unit includes a stage 117 on which the flow cell 109 is placed, and a driving motor (not shown) for driving the stage 117. The stage 117 is movable in directions along an X-axis and a Y-axis orthogonal to each other in the same plane. The stage 117 can also be moved in a Z-axis direction orthogonal to the XY plane by a driving motor different from the stage driving motor.

The temperature control unit adjusts a reaction temperature for a DNA fragment. The temperature control unit is disposed on the stage 117, and includes a temperature control substrate 118 for promoting a reaction between a DNA fragment to be analyzed and a reagent. The temperature control substrate 118 is implemented by, for example, a Peltier element.

The optical unit provides units for irradiating the analyzing area 123 of the flow cell 109 to be described below with excitation light and detecting fluorescence emitted from a DNA fragment. The optical unit includes a light source 107, a condenser lens 110, an excitation filter 104, dichroic mirrors 105 and 120, a band-pass filter 103, an objective lens 108, imaging lenses 102 and 121, and two-dimensional sensors 101 and 122. The excitation filter 104, the dichroic mirror 105, and the band-pass filter 103, which is also referred to as an absorption filter, are provided as a set in a filter cube 106. The band-pass filter 103 and the excitation filter 104 determine a wavelength region that allows fluorescence having a specific wavelength to pass.

A flow of irradiation with the excitation light in the optical unit will be described. The excitation light emitted from the light source 107 is condensed by the condenser lens 110 and enters the filter cube 106. Only a specific wavelength band of the entered excitation light is transmitted through the excitation filter 104. The transmitted light is reflected by the dichroic mirror 105 and condensed on the flow cell 109 by the objective lens 108.

Next, a flow of fluorescence detection in the optical unit will be described. The condensed excitation light excites a fluorescent substance, which is to be excited in the specific wavelength band, among four types of fluorescent substances incorporated into a DNA fragment immobilized on the flow cell 109. Fluorescence emitted from the excited fluorescent substance is transmitted through the dichroic mirror 105, only a specific wavelength band is transmitted through the band-pass filter 103, only a specific wavelength band is reflected by the dichroic mirror 120, and other wavelength regions are transmitted through the dichroic mirror 120. The light transmitted through the dichroic mirror 120 is imaged as a fluorescent spot on the two-dimensional sensor 101 by the imaging lens 102. The light reflected by the dichroic mirror 120 is imaged as a fluorescent spot on the two-dimensional sensor 122 by the imaging lens 121.

In the present embodiment, the number of types of fluorescent substances to be excited in a specific wavelength band is designed to be only one, and as will be described below, it is assumed that four types of bases can be identified according to types of the fluorescent substances. In addition, two sets of filter cubes 106 are prepared in accordance with wavelength bands of irradiation light and detection light, and these are sequentially switched, so that the four types of fluorescent substances can be sequentially detected. Transmission properties of the excitation filter 104, the dichroic mirrors 105 and 120, and the band-pass filter 103 in each filter cube 106 are designed such that the fluorescent substances can be detected with the highest sensitivity.

Similar to a normal computer, the computer 119 includes a processor (CPU), a storage device (various memories such as a ROM and a RAM), an input device (a keyboard, a mouse, and the like), and an output device (a printer, a display, and the like). The computer functions as a control processing unit that analyzes the fluorescent image detected and generated by the two-dimensional sensors 101 and 122 of the optical unit and performs base identification of each DNA fragment, in addition to the control for controlling the fluid delivery unit, the conveyance unit, the temperature control unit, and the optical unit described above. The control of the fluid delivery unit, the conveyance unit, the temperature control unit, and the optical unit described above, the image analysis, and the base identification may not necessarily be controlled and processed by one computer 119, and may be performed by a plurality of computers functioning as a control unit and a processing unit for the purpose of distributing a processing load, reducing a processing time, and the like.

(2) Decoding Method of DNA Base Sequence

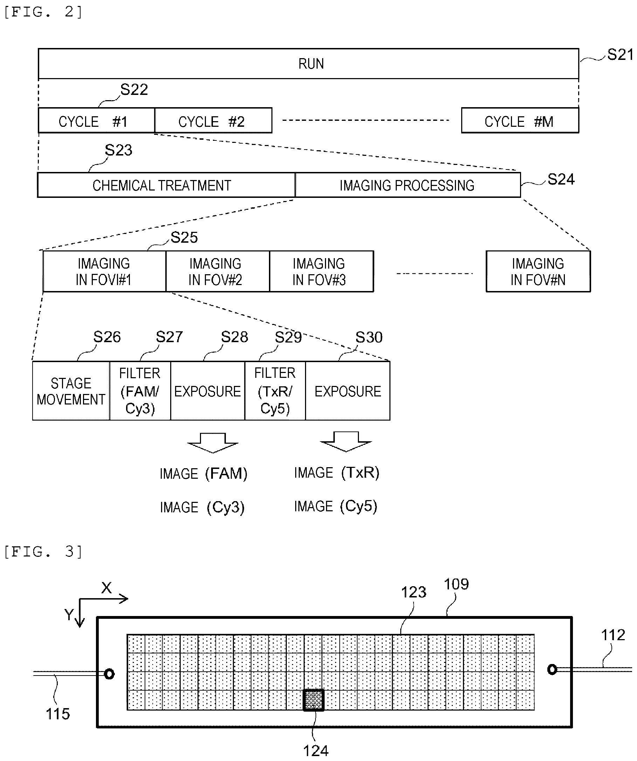

A method of decoding a DNA base sequence will be described with reference to FIGS. 2 to 4. As will be described below, it is assumed that colonies obtained by amplifying and densely arranging the same DNA fragment are disposed in a high density on the flow cell 109 in advance. The amplification of the DNA fragment may be performed using an existing technique such as emulsion PCR and bridge PCR.

FIG. 2 is a diagram showing processing steps for decoding a DNA base sequence. An entire run (Run) (S21) for decoding is performed by repeating cycle processing (S22) M times. M is a length of a base sequence to be obtained, and is determined in advance. Each cycle processing is processing for specifying a k-th (k = 1 to M) base, and is divided into a chemical treatment (S23) and imaging processing (S24) described below.

(A) Chemical treatment: Treatment for Extending Bases

In the chemical treatment (S23), the following procedures (i) and (ii) are performed.

(i) In the case of a cycle other than the first cycle, fluorescently labeled nucleotides (described below) in the previous cycle are removed from a DNA fragment and washed. A reagent for this purpose is introduced onto the flow cell 109 through the pipe 112. A waste liquid after washing is discharged to the waste liquid tank 116 through the pipe 115.
(ii) A reagent containing fluorescently labeled nucleotides flows to the analyzing area 123 on the flow cell 109 via the pipe 112. By adjusting a temperature of the flow cell by the temperature control substrate 118, an extension reaction occurs due to a DNA polymerase, and fluorescently labeled nucleotides complementary to DNA fragments on the colony are incorporated.

Here, the fluorescently labeled nucleotides are obtained by labeling four types of nucleotides (dCTP, dATP, dGTP, and dTsTP) with four types of fluorescent substances (FAM, Cy3, Texas Red (TxR), and Cy5), respectively. The respective fluorescently labeled nucleotides are described as FAM-dCTP, Cy3-dATP, TxR-dGTP, and Cy5-dTsTP. These nucleotides are complementarily incorporated into the DNA fragment, so that dTsTP is incorporated into the DNA fragment when an actual base of the DNA fragment is A, dGTP is incorporated in the case of the base C, dCTP is incorporated in the case of the base G, and dATP is incorporated in the case of the base T. That is, the fluorescent substance FAM corresponds to the base G, the fluorescent substance Cy3 corresponds to the base T, the fluorescent substance TxR corresponds to the base C, and Cy5 corresponds to the base A. The fluorescently labeled nucleotides are blocked at the 3'-terminal so as not to extend to the next base.

(B) Imaging Processing: Processing for Generating Fluorescent Image

The imaging processing (S24) is performed by repeating imaging processing (S25) for each field of view described below N times. Here, N is the number of the fields of view.

FIG. 3 is a diagram illustrating a concept of the field of view. When the entire analyzing area 123 is divided into N areas, a field of view (FOV) 124 corresponds to an individual area. The size of the field of view 124 is a size of an area that can be detected by the two-dimensional sensors 101 and 122 in one fluorescence detection, and is determined by the design of the optical unit. As will be described below, fluorescent images corresponding to the four types of fluorescent substances are generated for each field of view 124.

(B-1) Imaging Processing for Each Field of View

In the imaging processing (S25) in a field of view, the following procedures (i) to (iv) are performed.

(i) The stage 117 is moved such that the field of view 124 where fluorescence detection is performed is located at a position to be irradiated with the excitation light from the objective lens 108 (S26) . In this case, a focus position may be adjusted by driving the objective lens 108 in order to correct the deviation in a vertical direction caused by the movement of the stage 117.
(ii) The filter cube 106 is switched to a set corresponding to the fluorescent substance (FAM/Cy3) (S27).
(iii) By emitting the excitation light and simultaneously exposing the two-dimensional sensors 101 and 122, a fluorescent image (FAM) is generated on the two-dimensional sensor 101, and a fluorescent image (Cy3) is generated on the two-dimensional sensor 122 (S28).
(iv) The filter cube 106 is switched to a set corresponding to the fluorescent substance (TxR/Cy5) (S29).
(v) By emitting the excitation light and simultaneously exposing the two-dimensional sensors 101 and 122, a fluorescent image (TxR) is generated on the two-dimensional sensor 101, and a fluorescent image (Cy5) is generated on the two-dimensional sensor 122 (S30).

By executing the above processing, fluorescent images for the four types of fluorescent substances (FAM, Cy3, TxR, and Cy5) are generated for each field of view. In the fluorescent image, a signal of a fluorescent substance corresponding to the type of bases in the DNA fragment immobilized on the flow cell 109 appears as a colony on the image. That is, it is determined that a colony detected in a fluorescent image of FAM is the base A, a colony detected in a fluorescent image of Cy3 is the base C, a colony detected in a fluorescent image of TxR is the base T, and a colony detected in a fluorescent image of Cy5 is the base G.

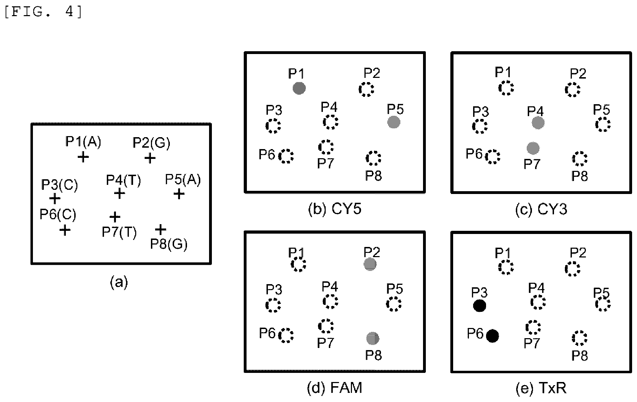

FIG. 4 is a diagram showing a concept of colonies on four types of fluorescent images in each field of view. As shown in (a) of FIG. 4, for example, in a certain field of view in a certain cycle, there are colons at eight positions P1 to P8, and the bases at the positions P1 to P8 are A, G, C, T, A, C, T, and G, respectively.
In this case, colonies are detected in accordance with the corresponding base type at the positions P1 to P8 in the fluorescent images for the four types of fluorescent substances (Cy5, Cy3, FAM, and TxR), as shown in (b) to (e) of FIG. 4. The positions of P1 to P8 are the same in the four fluorescent images. However, depending on the design of the optical unit, a difference in an optical path occurs for each wavelength, and thus, there is a possibility that the positions in the four fluorescent images are not exactly the same. Therefore, the colony positions on the four types of fluorescent images can be made the same by performing registration processing to be described below as necessary. However, when there is crosstalk in which the wavelength properties of filters of the respective fluorescent substances overlap each other, a colony of a certain base type is observed in two or more fluorescent images. The base type in this case can be identified by ROI images of the colony on the four types of fluorescent images, as will be described below. As described above, the base type of each colony detected in the field of view is determined.

(C) Repeating of Cycle Processing

By repeating the above cycle processing by the number of times corresponding to a length M of a desired base sequence, the base sequence having the length M can be determined for each colony.

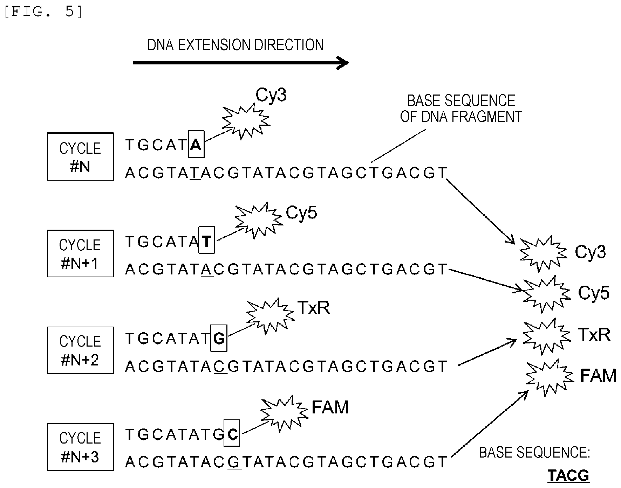

FIG. 5 is a diagram showing a concept of determination of the base sequence. As shown in FIG. 5, when each colony (a DNA fragment having a base sequence ACGTATACGT ...) is extended by the amount of one base by a chemical treatment (S23) in a certain cycle (#N), for example, Cy3-dATP is incorporated in the colony. The fluorescently labeled nucleotide is detected as a colony on the fluorescent image of Cy3 in the imaging processing. Similarly, in a cycle (#N+1), the fluorescently labeled nucleotide is detected as a colony on the fluorescent image of Cy5. In a cycle (#N+2), the fluorescently labeled nucleotide is detected as a colony on the fluorescent image of TxR. In a cycle (#N+3), the fluorescently labeled nucleotide is detected as a colony on the fluorescent image of FAM. By the cycle processing from the cycle #N to the cycle #N+3, the base sequence in the colony is determined as TACG.

(3) Details of Base Calling Processing

As described above, the DNA fragment to be detected is observed as spots on the four fluorescent images, and a base is called in each cycle.

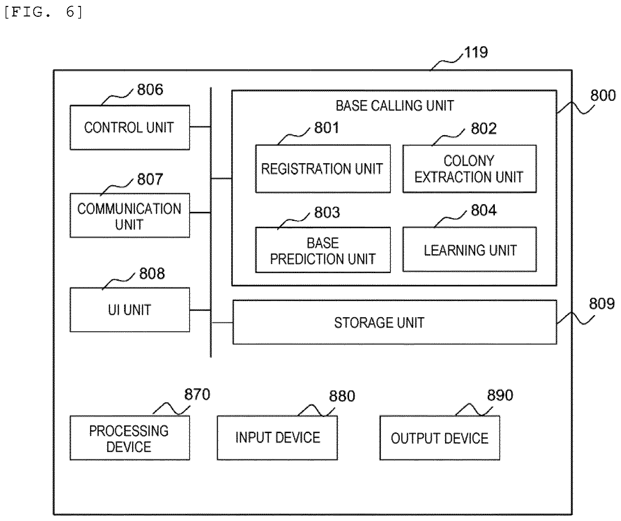

FIG. 6 is a block diagram showing a functional configuration of the computer 119 in the nucleic acid analyzer 100. The computer includes a control unit 806 that controls the fluid delivery unit, the conveyance unit, the temperature control unit, and the optical unit described above, and a base calling unit; a communication unit 807 that exchanges control commands and image data between the computer 119 and the apparatus; a UI unit 808 that presents a screen to a user and receives inputs from the user; a storage unit 809 implemented by a memory, a hard disk, and the like; and a base calling unit 800 that outputs a base sequence. The base calling unit 800 includes a registration unit 801, a colony extraction unit 802, a base prediction unit 803, and a learning unit 804. Hereinafter, the base calling processing performed in the base calling unit 800 will be described.

In the present embodiment, it is assumed that the control unit 806, the communication unit 807, the UI unit 808, and the base calling unit 800 are implemented by software. That is, programs for performing calculation and processing of the units are stored in a storage device of the computer 119, and a processing device 870 executes these programs to perform processing in cooperation with hardware such as the input device 880, the output device 890, and the storage unit 809. As described above, the control unit 806, the communication unit 807, the UI unit 808, and the base calling unit 800 may be implemented by hardware instead of software.

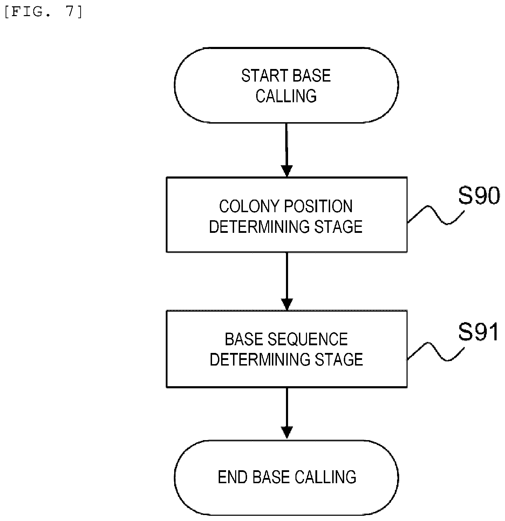

FIG. 7 is a diagram showing a flow of the base calling processing. The base calling processing is performed in two stages including a colony position determining stage (S90) and a base sequence determining stage (S91).

(A) Colony Position Determining Stage

The colony position determining stage (S90) is performed in the base calling unit 800. In the present embodiment, a colony as a base calling target is determined based on the images from the first cycle to the N-th cycle in the colony position determining stage (S90).

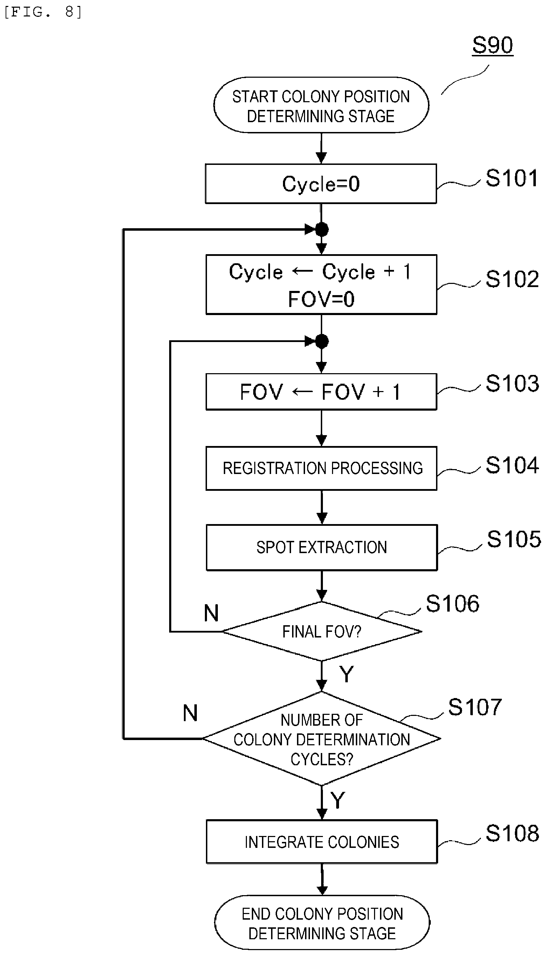

The flow of the colony position determining stage (S90) will be described with reference to FIG. 8. The registration unit 801 acquires fluorescent images of four colors in a first FOV of the first cycle through steps S101, S102, and S103, and performs registration processing with a reference image (S104). The registration processing will be described below.

(A-1) Registration of Images

As described above, since the nucleic acid analyzer 100 acquires four fluorescent images by the two sensors 101 and 122, a positional deviation occurs between the fluorescent images. When imaging is repeatedly performed in the same field of view in the cycles, the stage 117 is moved to change the field of view in the cycles. Therefore, for the same field of view, a positional deviation due to a control error during the movement of the stage occurs between different cycles.

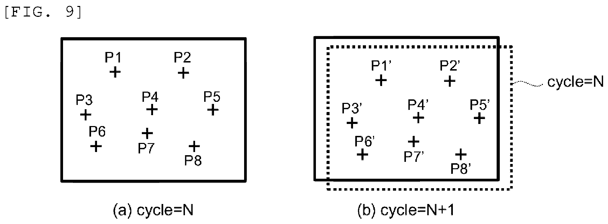

FIG. 9 is a diagram showing a concept of a positional deviation between cycles. As shown in FIG. 9, for a certain field of view (FOV), there is a possibility that an imaging position is deviated due to a stage control error between the N-th cycle shown in (a) of FIG. 9 and the (N+1)-th cycle shown in (b) of FIG. 9. Therefore, the DNA fragment positions (P1 to P8) on a fluorescent image in the N-th cycle are respectively detected as different positions (P1' to P8') on a fluorescent image in the (N+1)-th cycle. However, these spots are all derived from the same DNA fragment. Therefore, in order to determine the base sequence of each colony, it is necessary to correct the positional deviation between the colonies detected in the fluorescent images.

In order to correct the positional deviation, it is necessary to register the fluorescent images relative to a common reference image. Here, the reference image is a common image used for the position coordinate system of a colony. For example, if a position of the colony is known as design data, the reference image may be created based on the known colony position. As an example, an image having luminance according to a two-dimensional Gaussian distribution of dispersion defined in advance and corresponding to a colony size with a colony position (x, y) as the center may be created. Alternatively, the reference image may be created based on any one of actually captured images. As an example, an image of each field of view in the first cycle may be set as a reference image, and an image of each field of view in the second and subsequent cycles may be registered with the reference image.

A known matching technique can be applied to the registration between images. As an example, an image obtained by cutting out a part of the reference image is set as a template image t (x, y), a cross-correlation function m (u, v) between the template image t (x, y) and a target image f (x, y) obtained by cutting out a part of an input image is determined, and S_1 = (u, v) giving a maximum value of the cross-correlation function m (u, v) is set as the positional deviation amount. Here, an example of t (x, y) is an image of 256 pixels × 256 pixels at a center of the reference image. Similarly, an example of f (x, y) is an image of 256 pixels × 256 pixels at a center of the input image. For the calculation of the positional deviation amount, a normalized cross-correlation considering a difference in brightness may be used instead of the cross-correlation function, or a correlation limited to a phase may be used. In the case of detecting the deviation amount of an angle between the images, the above-described cross-correlation or phase-only correlation can be applied to an image in which an angle direction is transformed to a horizontal direction by performing polar coordinate transformation on the image.

In addition, the positional deviation amount may be determined at a plurality of points in accordance with a degree of distortion of the image.

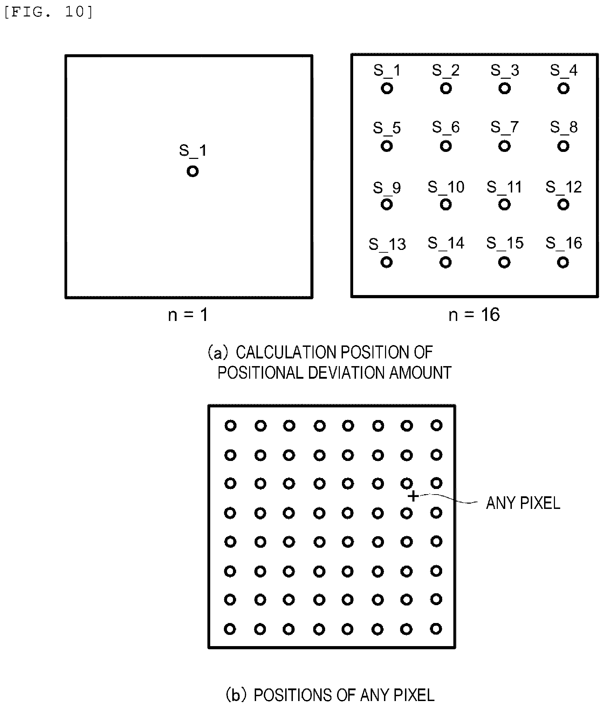

FIG. 10 shows the concept. For example, when there is no distortion in the image and the same positional deviation relative to all the pixels, that is, only a uniform deviation caused by the stage can be assumed, the positional deviation amount $S\_1$ (u, v) shown on a left side of (a) of FIG. 10 can be applied.

On the other hand, for example, when there is distortion in the image and the positional deviation amount varies depending on the position in the image (when the flow cell 109 is deformed by being heated and the positional deviation is not uniform), as shown on a right side of (a) of FIG. 10, positional deviation amounts are determined at a plurality of (n) points in the image, and the positional deviation amounts $S\_1, S\_2, ... S\_n$ at the plurality of points are determined. In the calculation of the positional deviation amount at the points, images centered on the positions of the points in the reference image and the input image are cut out, and the images are used as the template images and the target images, respectively, and a positional deviation amount at which the correlation is maximized may be calculated as described above. Then, based on the obtained n positional deviation amounts, a positional deviation amount at any pixel position can be formulated by determining, for example, a coefficient of affine transformation or polynomial transformation according to the least square method (see (b) of FIG. 10). The transformation may be calculated in both directions. That is, the mutual transformation between a coordinate system of the input image and a coordinate system of the reference image may be defined.

(A-2) Spot Extraction Processing

In FIG. 8, the colony extraction unit 802 extracts spot positions indicating a colony from registered fluorescent images (S105). As an example of a method of determining the spot position, there is a method of performing a predetermined threshold value determination on an input image, dividing the input image into a spot area and a non-bright spot area, and searching for a local maximum value from the spot area.

Prior to the spot extraction processing, noise may be removed from the input image by a low-pass filter, a median filter, or the like. In addition, background correction processing may be performed on the assumption that luminance unevenness occurs in the image. As an example of the background correction processing, a method may be used in which an image obtained by imaging an area where no DNA fragment is present in advance is set as a background image, and the background image is subtracted from the input image. Alternatively, a high-pass filter may be applied to the input image to remove a background component that is a low-frequency component.

It should be noted that, although the colony is included in any one of the four types of fluorescent images, there is also a possibility that spots derived from one colony are included in a plurality of fluorescent images due to an influence of crosstalk as described above. Spots on different fluorescent images, which are determined to be close to each other by the registration, may be integrated as described below.

As described above, in the present embodiment, the colony position is determined using the images from the first cycle to the N-th cycle. Here, N is referred to as the number of colony determination cycles. N may be about 1 to 8.

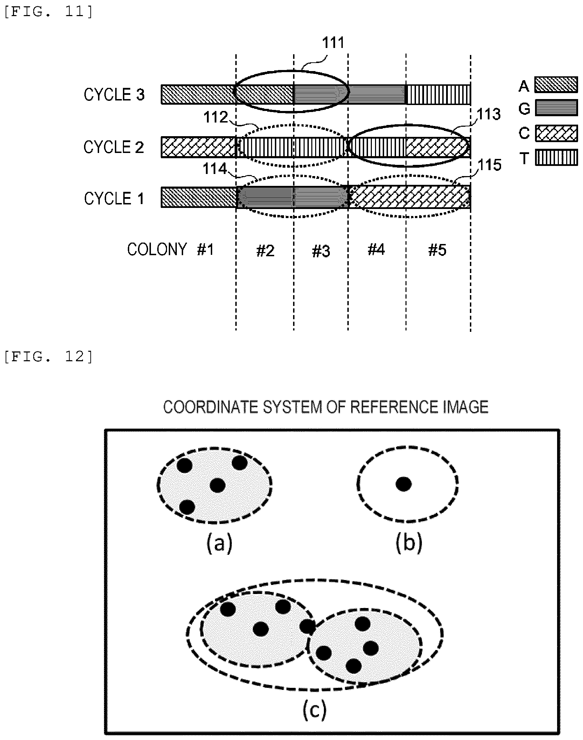

FIG. 11 shows an advantage of extracting a colony by using a plurality of cycles. This figure schematically shows bases of the side-by-side adjacent colonies #1 to #5 in three cycles. When adjacent colonies have the same base in the same cycle, these colonies are adjacent to each other in one fluorescent image, and thus it may be difficult to distinguish the colonies from each other. Therefore, by referring to a plurality of cycles, the colonies at positions where bases are different can be easily distinguished.

In the example of FIG. 11, the colony #2 and the colony #3 (114) having the same base and the colony #4 and the colony #5 (115) having the same base are adjacent to each other in the cycle 1, and thus it is difficult to identify these colonies in a fluorescent image of only the cycle 1. In the cycle 2, the bases of colony #4 and colony #5 (113) among these colonies are different from each other, so that identification is facilitated. Similarly, in the cycle 3, the colony #2 and the colony #3 (111) have different bases, so that the identification is facilitated. In this way, the identification of the colony can be enhanced by using a plurality of cycles.

In FIG. 8, the colony extraction unit 802 repeats the spot extraction processing (S105) for each field of view in each cycle (S106). In the case of a final field of view in one cycle, the process proceeds to the next cycle (S102), and proceeds to the first field of view (S103), and the registration processing (S104) and the spot extraction (S105) are repeated. When the number of cycles from the first cycle reaches the number of colony determination cycles N (S107), processing of integrating colonies obtained up to the N-th cycle (S108) is performed.

(A-3) Colony Integration Processing

In the colony integration processing (S108), the colony extraction unit 802 integrates the spots extracted from the fluorescent images in the N cycles, which are transformed into a coordinate system of a reference image by the registration.

FIG. 12 shows a concept of the colony integration. As shown in this figure, even in the case of the same colonies, the colonies are not accurately disposed at the same coordinates in the coordinate system of the reference image due to an error in the registration calculation. Therefore, in the colony integration processing, mutually adjacent colonies within a certain distance may be regarded as one colony ((a) of this figure), or when there is only one colony, the one colony may also be regarded as one effective colony ((b) of this figure). When a size of an adjacent colony exceeds a certain threshold value, the adjacent colony may be divided into two colonies. A centroid of new colonies that are integrated may be recalculated. For example, an existing clustering technique such as a k-means method may be applied to these integration algorithms.

By the above processing, it is possible to correct the positional deviation between the four types of fluorescent images and the positional deviation between the images in the plurality of cycles, and the colony position in the images is determined.

(B) Base Sequence Determining Stage

Next, details of the processing of the base sequence determining stage (S91) in the base calling processing of FIG. 7 will be described. In this stage, with respect to a colony determined in the above-described colony position determining stage (S90), a base sequence is determined by calling the bases of all cycles.

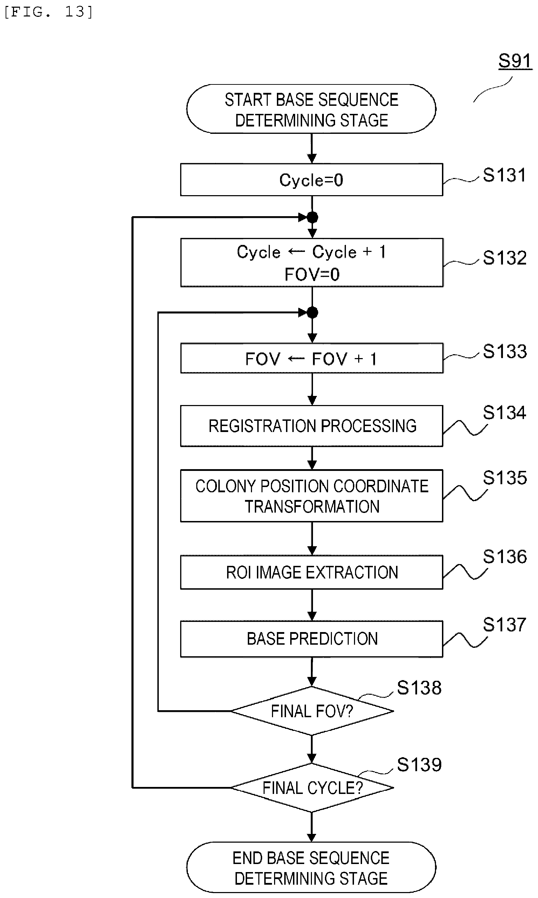

FIG. 13 shows a flow of the base sequence determining stage (S91). Through steps S131, S132, and S133, the processing proceeds to the first FOV of the first cycle, and thereafter, the following processing is performed on each FOV.

(B-1) Registration Processing (S134)

The registration unit 801 performs registration of the four fluorescent images in the FOV to be processed relative to the reference image. The method is the same as the method described in (A-1). In this case, since the registration has already been performed in the images up to the number of the colony position determination cycles in the previous stage, the result of the registration at that time may be used.

(B-2) Colony Position Coordinate Transformation (S135)

The colony extraction unit 802 transforms coordinates of all the colonies on the reference coordinate system determined in the previous stage into a coordinate system of the four fluorescent images to be processed. For the transformation, the result of the registration in step S134 is used. Accordingly, colony positions on the fluorescent images are obtained.

(B-3) ROI Image Extraction (S136)

The colony extraction unit 802 extracts an ROI (region of interest) image centered on the colony position on each fluorescent image.

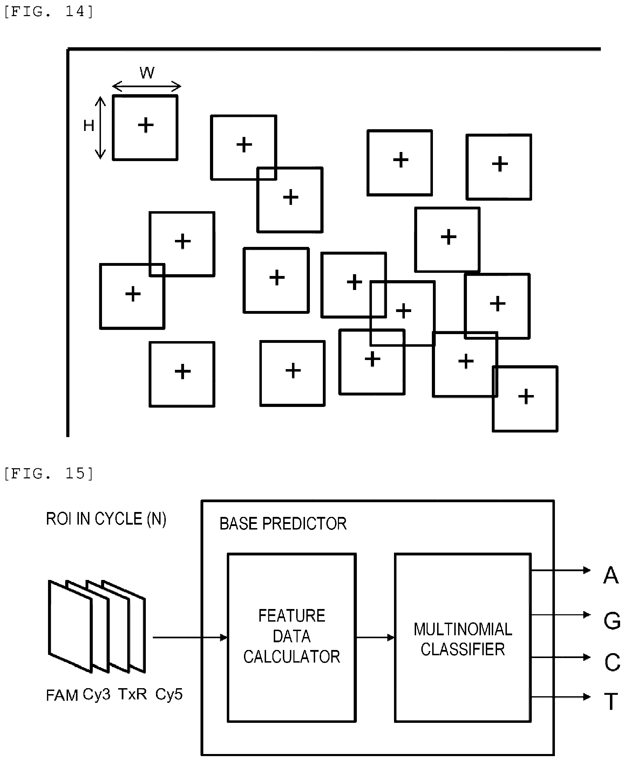

FIG. 14 shows a concept of the ROI image extraction. The symbol "+" represents a center of a colony position on a fluorescent image, and an area of W pixels × H pixels centered on "+" is extracted. Here, W and H are appropriately determined in advance in accordance with a size of the colony and a resolution of the image. It is desirable that a colony adjacent to the colony is not reflected as much as possible. Prior to the ROI image extraction, pixel values of a fluorescent image may be normalized in accordance with base prediction described below.

By extracting not only spots on the fluorescent image but also surrounding pixels, it is possible to obtain accompanying information when acquiring the fluorescent image, such as positional deviation, defocus, and crosstalk of the image. By increasing the information amount of the fluorescent image in this way, it is possible to improve the prediction accuracy of the base predictor using machine learning as described below.

(B-4) Base Prediction (S137)

The base prediction unit 803 receives a set including individual ROIs of the fluorescent images of the four colors as inputs to perform base prediction.

FIG. 15 shows an example of a base predictor in the base prediction unit 803. The base predictor includes a feature data calculator and a multinomial classifier. The feature data calculator calculates feature data based on input images, and the multinomial classifier classifies the input images into any one of A, G, C, and T based on the feature data.

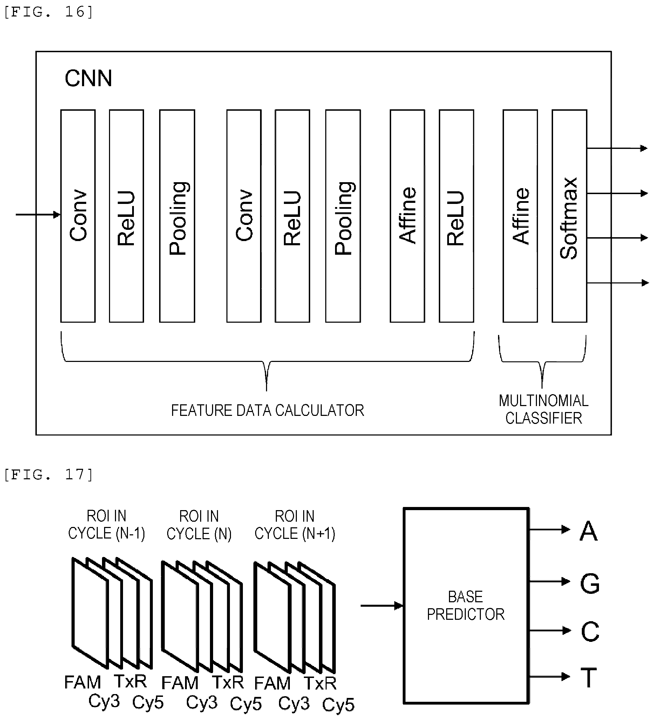

FIG. 16 shows a configuration using a convolutional neural network (CNN) as an example of such a base predictor. In the Convolution layer (Conv in the figure), the following filter operation is performed on an input image. The CNN is an example of a supervised learning neural network.

[Formula 1]

$$u_{ijm} = \sum_k \sum_p \sum_q I_{i+p,j+q,k} h_{pqkm} + b_{ijm} \qquad \cdots (1)$$

Here, I represents an input image, h represents a filter coefficient, and b represents an addition term. Further, k represents an input image channel, m represents an output channel, i and p represent horizontal positions, and j and q represent vertical positions.

The ReLU layer applies the following activation function to an output of the above-described Convolution layer.

[Formula 2]

$$ReLU(x) = \max(0, x) \qquad \cdots (2)$$

As the activation function, a nonlinear function such as a tanh function, a logistic function, or a rectified linear function (ReLU) may be used.

The Pooling layer slightly reduces position sensitivity of the feature data extracted from the Convolution layer and the ReLU layers, so that the output is unchanged even when a position of feature data in an image slightly changes. Specifically, a representative value is calculated based on a partial area of the feature data with a constant step size. For the representative value, an average value or the like is used as a maximum value. There is no parameter that changes due to learning in the Pooling layer.

The Affine layer is also called a fully connected layer, and defines weighted connection from all units of an input layer to all units of an output layer. Here, i represents an index of the unit of the input layer, and j represents an index of the unit of the output layer. w represents a weight coefficient between them, and b represents an addition term.

[Formula 3]

$$u_j = \sum_i w_{ji} x_i + b_j \qquad \cdots (3)$$

In the CNN, the result obtained by repeatedly executing the Convolution layer, the ReLU layer, and the Pooling layer and passing through the Affine layer to the ReLU layer is image feature data. Based on the image feature data obtained in this way, a multinomial classification, that is, base determination of A, G, C, and T is performed.

As an example of the multinomial classification method, the image feature data is further subjected to Affine layer processing in the present embodiment, and logistic regression using the following softmax function is applied to the result.

[Formula 4]

$$y_k = e^{x_k} / \sum_j e^{x_j} \qquad \cdots (4)$$

Here, y represents a value indicating the likelihood of a label (base herein) corresponding to an output unit k. In the present embodiment, the output unit k corresponds to the likelihood of a base type k, and a base type having the largest likelihood is set as the final classification result.

The filter coefficient and the addition term of the Convolution layer and the weight coefficient and the addition term of the Affine layer as described above are determined in advance by training processing executed by the learning unit 804 as described below. These coefficients are stored as predictor parameters in the storage unit 809. During the base prediction processing, the base prediction unit 803 may appropriately acquire the coefficients from the storage unit 809.

The base prediction processing (S137) as described above is performed on all the FOVs in all the cycles (S138, S139), so that the base sequences in all the FOVs in all the cycles are determined (the base sequence determining stage S91 is ended).

As described above, in the nucleic acid analyzer according to the first embodiment, ROI images of fluorescent colors obtained by performing the registration and the colony extraction are received as inputs, the feature data of the ROI images is calculated, and the base prediction is performed based on the feature data. Therefore, base prediction robust to positional deviation and defocus of an image can be implemented.

[Second Embodiment]

A second embodiment will be described with reference to FIG. 17. In the second embodiment, as shown in FIG. 17, the base predictor described above receives ROI images in cycles previous or next to a certain cycle (N-th cycle) as inputs in addition to inputs of ROI images in the certain cycle. It is assumed that these ROI images in cycles previous and next to the certain cycle are registered in advance and are images derived from the same colony. In FIG. 17, all the ROI images have the same size, and each fluorescent image is input to correspond to one channel. That is, a 12-channel ROI image is received as an input in FIG. 17.

As an advantage of receiving the ROI images in cycles previous and next to the certain cycle as inputs, the base prediction can be performed in consideration of the influence of fading between cycles.

The fading causes a deviation in a pace of the extension reaction due to imperfection in a chemical reaction of a DNA fragment in each cycle, and not only a signal derived from bases in each cycle but also signals derived from bases in cycles previous and next to the cycle are mixed. It is known that such fading exists at a certain rate in each cycle, and such an influence is accumulated as the cycle progresses, which is a cause of a decrease in an accuracy of base identification.

As described above, during the training and during the prediction, a fluorescent signal in each cycle is mixed with fluorescent signals derived from the same colony in the cycles previous and next to the cycle, so that the base predictor can perform base prediction in consideration of fading by using a model for predicting a base based on the inputs including the ROI images in a previous cycle and a next cycle. The ROI images in only the previous cycle and the next cycle are received as inputs in this figure, and ROI images in two or more previous cycles and two or more next cycles may be received as inputs. Alternatively, images in one of the previous cycle and the next cycle may be received as inputs.

As described above, in the nucleic acid analyzer according to the second embodiment, a plurality of ROI images in cycles previous and next to the cycle to be predicted are added to the input image, and base prediction is performed, so that highly accurate base prediction can be implemented in consideration of the influence of fading.

[Third Embodiment]

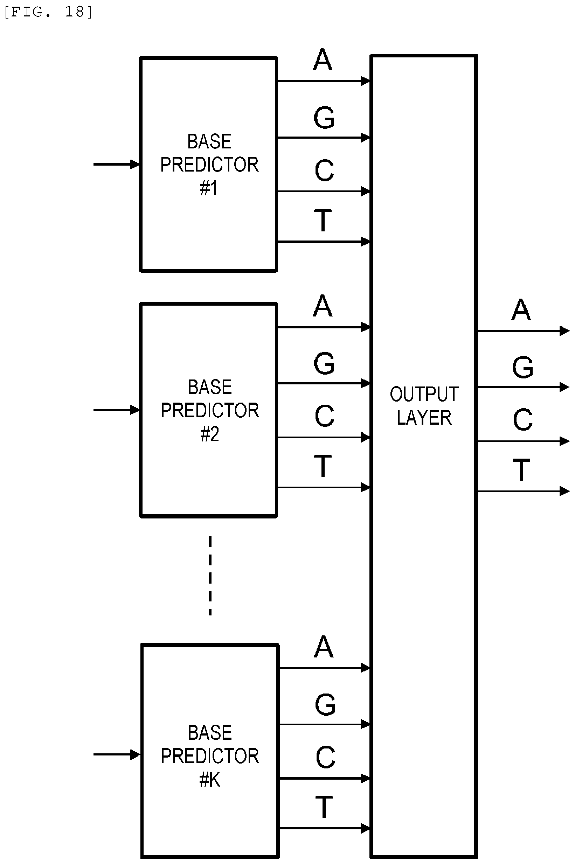

A third embodiment will be described with reference to FIG. 18. In the third embodiment, as shown in FIG. 18, the base prediction unit 803 performs base prediction by combining a plurality of base predictors described above. In this figure, the respective base predictors receive ROI images of the same size as inputs, and different base prediction parameters are set to the respective base predictors. It is assumed that these different base prediction parameters are determined in advance by training as will be described below under different conditions. Here, the different conditions refer to different devices, different room temperatures, different cycles, or the like, and may be determined in consideration of variations in the captured images of the RUN.

In an output layer of this figure, the final base likelihood is output from the outputs (the likelihoods of the bases) of the plurality of base predictors determined under such different conditions. With such a configuration, base prediction with higher reliability in consideration of various conditions can be performed. As the processing of the output layer, the maximum value of all the base predictors may be output, or an average of the likelihoods of the bases or the weighted sum may be output.

It should be noted that the base predictors may have different network structures of CNN, different numbers of cycles previous and next to a certain cycle for an ROI image received as an input, and different ROI sizes. In addition, the feature data extraction methods and the multinomial classification algorithms may be different.

As described above, in the nucleic acid analyzer according to the third embodiment, a plurality of base predictors determined under different conditions are used. Therefore, robust and more accurate base prediction relative to various conditions can be implemented.

[Fourth Embodiment]

In a fourth embodiment, an example of a training method of the base predictor in the base prediction unit 803 described in the first embodiment will be described. The fourth embodiment describes, as an example, a configuration shown in FIG. 6 of the first embodiment, in which the learning unit 804 is added to the base calling unit 800. However, the training of the base predictors may be individually performed by different devices. In this case, the learning unit 804 in FIG. 6 may be omitted.

Details of Training Processing

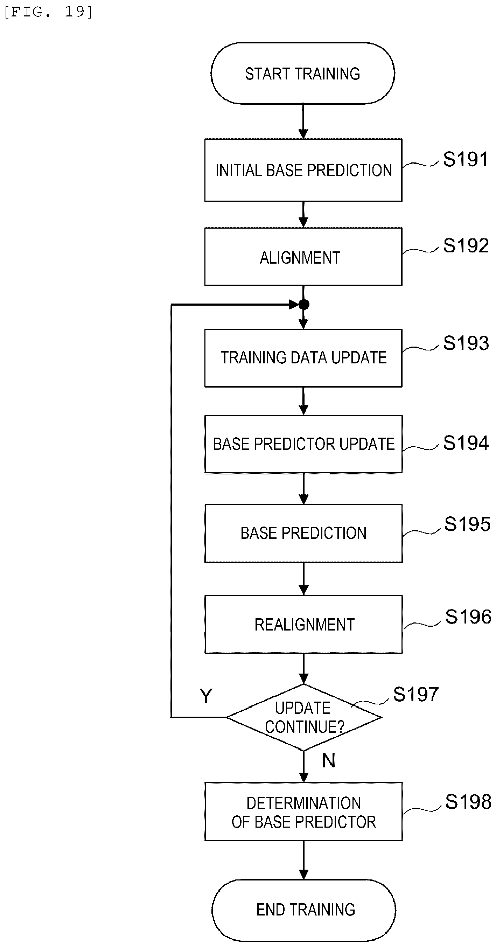

FIG. 19 shows a flow of a training processing of the base predictor in the learning unit 804.

(A) Initial Base Prediction (S191)

In the initial base prediction (S191), an initial value of a base sequence for each colony determined in the colony position determining stage (S90) is output. The initial base prediction may be a prediction based on a simple rule in which a base corresponding to a fluorescent color at which the luminance of the colonies is maximized is selected from the outputs of the base sequences. Alternatively, the initial base prediction may be implemented by setting initial prediction parameters using the base predictor (for example, the base prediction unit 803 in an initial setting state) described in the first embodiment. In this case, the correct or incorrect base sequence is determined based on the alignment processing with the reference sequence as will be described below, so that the initial base prediction is desired to have an accuracy at which a certain number of base sequences are aligned.

(B) Alignment Processing (S192)

The alignment processing is performed on the base sequence obtained by the initial base prediction (S192). The alignment processing refers to processing of associating the base sequences of all the obtained colonies with the reference sequence.

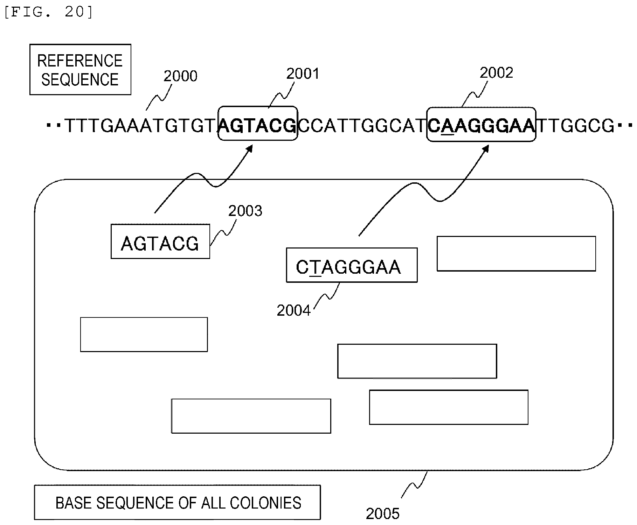

FIG. 20 is a conceptual diagram of the alignment processing. The reference sequence is a known correct sequence corresponding to a DNA sample measured by a nucleic acid analyzer. The reference sequence used here may be a widely published genome sequence or a correct sequence attached to a commercially available sample. As an algorithm of the alignment, a search based on a known method such as Burrows-Wheeler Transform may be used.

FIG. 20 shows a situation in which base sequences 2003 and 2004 of a certain colony in a set 2005 of the base sequences of all the colonies are aligned with a partial sequence 2001 and a partial sequence 2002 in a reference sequence 2000, respectively. For the base sequence aligned in this manner, it can be determined that a base that matches the reference sequence is correct, and a base that does not match the reference sequence is incorrect. In this figure, the base sequence 2003 and the partial sequence 2001 match each other, so that all the bases in the base sequence 2003 are determined to be correct. With reference to the partial sequence 2002, it can be determined that the second base from the head in the base sequence 2004 is incorrect, and the other bases therein are correct. Among the base sequences output from the base prediction unit 803, a sequence that has not been aligned is not determined to be correct or incorrect.

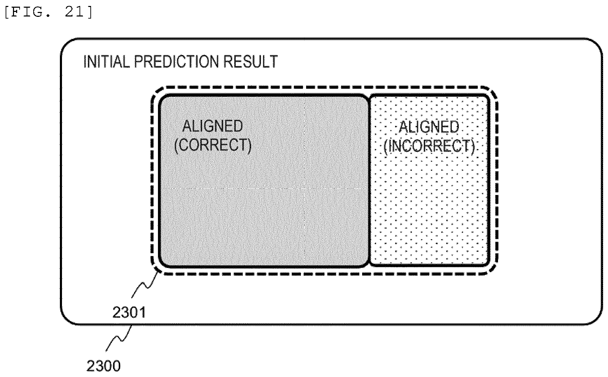

FIG. 21 shows a relation between a set of bases of the colonies and a set of bases of aligned colonies. A set 2300 is a set of bases estimated in all cycles by the base prediction unit 803 for all the colonies obtained by the colony extraction unit 802 of the first embodiment. A set 2301 is a set of aligned colonies as a result of performing alignment processing on the set 2300. The set 2301 can be further divided into correct bases and incorrect bases as shown in this figure.

The following alignment evaluation indexes are calculated for the alignment results obtained in this way and stored in the storage unit 809.

(1) Alignment Rate: a proportion of the number of aligned colonies to the number of all extracted colonies
(2) Correct base rate (or incorrect base rate): a proportion of the number of correct bases (or the number of incorrect bases) to the number of all the bases of the aligned colonies

(C) Training Data Update (S193)

The training data is generated using, as one piece of correct information, a combination of a fluorescent image corresponding to each base of a base sequence aligned in step S192 (or S196) and a correct base indicated by the reference sequence (S193).

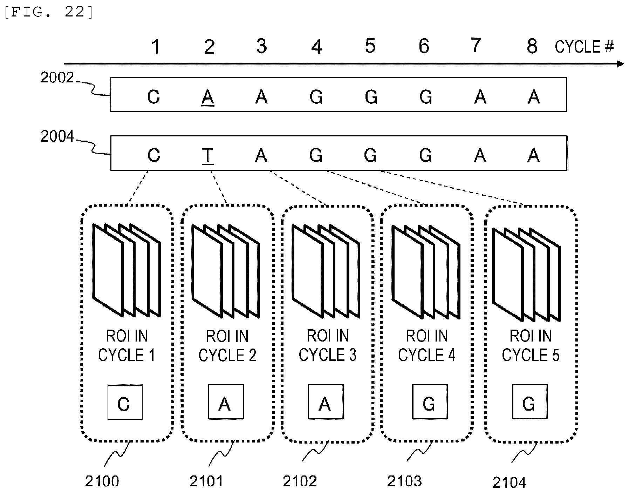

FIG. 22 shows a concept of the correct information in the training data. As an example, FIG. 20 shows an example of correct information of the base sequence 2004 aligned with the partial sequence 2002 of the reference sequence. A sequence position of the base sequence 2004 corresponds to a cycle, and each base is estimated from an ROI image in each cycle at a colony position. As indicated by 2100 to 2104 in FIG. 22, the correct information includes a combination of ROI images corresponding to respective bases and the correct base information indicated by the partial sequence 2002. The correct information 2100, 2102, 2103, and 2104 indicates that the predicted bases are correct.

The correct information 2101 indicates that the prediction result that the predicted base is T (on the base sequence 2004) is incorrect, and correct information in which the correct base "A" shown by the reference sequence and the ROI image are combined can be generated. The ROI image of each colony may be stored as a set of link information of a fluorescent image and position information of a colony on each fluorescent image. When the information is input to the base predictor, an ROI image can be acquired from these pieces of information.

In the present embodiment, information on a sequence aligned in this way may include both information on a correctly predicted base and information on an incorrectly predicted base. In particular, an incorrect base is estimated as an ROI image for which base prediction is inherently difficult, so that an improvement in a performance of the base predictor can be expected by including correct information of the incorrect base in the training data.

In a case where training data already exists, correct information of a base that does not exist in the existing training data is added to the training data.

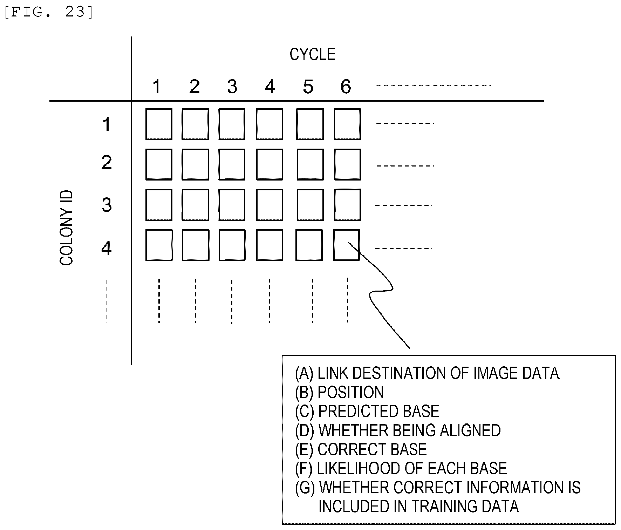

FIG. 23 is a conceptual diagram of update of the training data. An information table as shown in this figure is stored in the storage unit 809 for the bases of all the colonies in all the cycles. Contents of the information table may include, for example, the following information.

(a) A link destination of each piece of fluorescent image data (which is common to all the colonies, and thus may be held for each cycle)
(b) Positional information of colonies in each image
(c) Predicted base
(d) Whether being aligned
(e) Correct base (in the case of being aligned)
(f) Likelihood of each base
(g) Whether correct information is included in the training data

Referring to (g) above, if correct information of the base is not included in the training data, the correct information of the base is added to the training data. At this time, the contents of (c), (d), and (f) may be updated in accordance with the base prediction result described below as necessary.

(D) Base Prediction Unit Update (S914)

In this way, the training is performed using newly generated or updated training data, and the parameters for the base predictor are updated (S194). A known machine learning algorithm can be applied to the training. In the case of the Convolutional Neural Network described in the first embodiment, the known backpropagation is applied to determine the filter coefficient and the addition term in the Convolutional layer and the weight coefficient and the addition term in the Affine layer. In this case, a cross entropy error function may be used as an error function.

The coefficient at the start of training may be initialized randomly for the first time, or a pre-training method such as a known self-encoder may be applied. If the update of the base predictor itself in step S194 is the second or later, the predictor parameters determined last time may be used.

For the calculation of the predictor parameters described above, it is possible to use a method of updating the predictor parameters to minimize the error function by repeatedly calculating the predictor parameters by a predetermined number of iterations (number of epochs) using a known method such as a gradient descent method. A learning coefficient for updating the predictor parameters may be appropriately changed by a known method such as AdaGrad or Adadelta.

In the calculation of a gradient of the error function for updating the parameter described above, the gradient may be calculated based on a sum of the errors relative to all the data by the gradient descent method, or the predictor parameters may be updated by randomly dividing all the data into a set including a predetermined M pieces of data called mini-batches and calculating a gradient for each mini-batch by a known stochastic gradient descent method. In the stochastic gradient descent method described above, the influence of data bias may be reduced by shuffling data for each epoch.

In the training described above, a part of the training data may be separated as verification data, and the base prediction performance based on the predictor parameters trained using the verification data may be evaluated. The prediction performance based on the verification data may be visualized for each epoch. As an index of the prediction performance, prediction accuracy indicating a proportion of correct prediction, an error rate opposite to the prediction accuracy, a value (loss) of an error function, or the like may be used. The predictor parameters obtained by training in this manner are applied to the base predictor. In this case, as will be described later, the final determination on whether to adopt the latest predictor parameters is performed in step S199, so that the previous predictor parameters before the update (before training in step S194) are stored in the storage unit 809.

(E) Base Prediction (S195)

The base prediction unit 803 performs base prediction for all the colonies using the predictor parameters obtained in step S194, thereby outputting the base sequences of all the colonies. The base prediction according to the first embodiment is applied to this prediction.

(G) Realignment Processing (S196)

The learning unit 804 performs realignment processing on the base sequences obtained in step S195. The alignment processing is exactly the same as step S192 except that the input base sequence is different, and thus detailed descriptions thereof will be omitted.

(F) Determination of Update Continuation (S197)

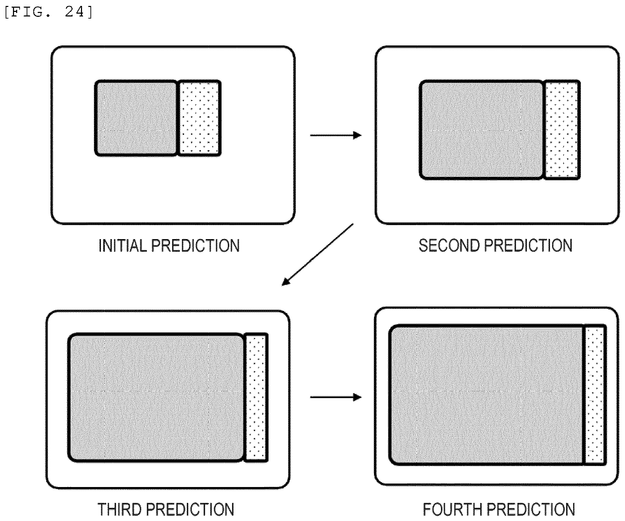

Based on the alignment rate and the correct base rate obtained in step S196, it is determined whether to continue or end the update processing of the predictor parameters described above.
As shown in FIG. 24, when repeating the above-described update processing of the predictor parameters basically, it is considered that the alignment rate and the correct base rate gradually increase, an increase rate gradually decreases and eventually saturates, or the training fails and the increase rate becomes negative.

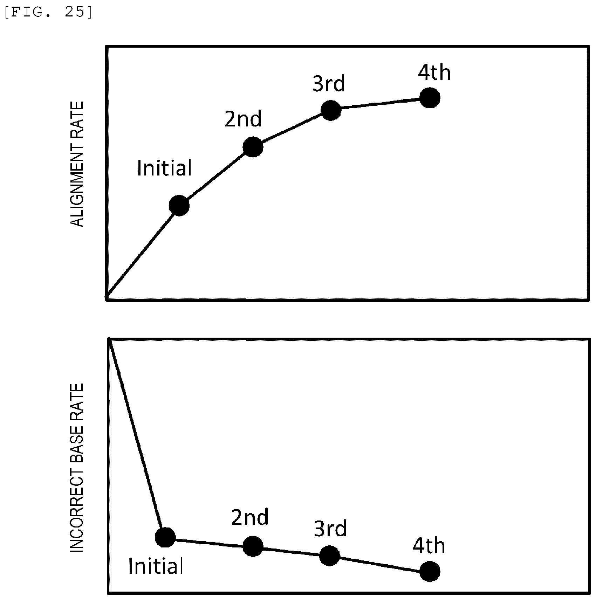

FIG. 25 shows that the alignment rate and the incorrect base rate are plotted for each number of times. Therefore, as a method of determining whether the update of the predictor parameters is continued, a determination may be used in which a determination threshold value may be provided for each of the increase rate of the alignment rate and the increase rate of the correct base rate, and the update of the predictor parameters may be ended when these increase rates fall below the threshold values.
When the update of the predictor parameters is continued, the processing returns to step S193, and the training data is updated using the correct information of the aligned colonies obtained in step S196.

(G) Determination of Base Prediction Unit (S198)

When the update of the predictor parameters is ended in step S198, one optimal predictor parameter is selected from among the predictor parameters obtained by repeating the update, including the initial base prediction (S191), and a base predictor is determined (S198).
Examples of a criterion for selecting the optimal parameter include the maximum alignment rate described above and the maximum correct rate. In this case, as the alignment rate increases, a base which is difficult to predict is more likely to be aligned, and thus the parameters may be determined based on such a criterion that a weighted sum of the alignment rate and the correct rate is maximum.
As described above, in the nucleic acid analyzer according to the fourth embodiment, a base sequence is generated by using the base predictor in the initial state for the captured image set given for training, the training data is updated by extracting the correct information from the colonies aligned by the alignment processing between the base sequence result and the reference sequence, and the predictor parameters are trained by using the training data. By repeating such processing, high-quality training data is extracted from the captured image set for training and applied to the training of the base predictor, so that the accuracy in the base identification can be improved.

[Fifth Embodiment]

A fifth embodiment is an example of training parameters of a base predictor in which the ROI image in the cycle to be subjected to base estimation and the ROI images in a plurality of cycles previous and next to the cycle, which are described in the second embodiment, are added to a channel of an input image.
In the present embodiment, the ROI images to be added to the training data as the correct information do not include the ROI images in one cycle (four channels) as described in FIG. 22, but include the ROI images in previous and next cycles. Other training methods are the same as those of the fourth embodiment.
As described above, in the fifth embodiment, training data obtained by adding the plurality of ROI images in cycles previous and next to the cycle to be predicted to the input images is generated, and predictor parameters are trained, so that a highly accurate base prediction can be implemented in consideration of the influence of fading.

[Sixth Embodiment]

In a sixth embodiment, an ROI image obtained by applying image processing to the ROI image included in the training data is added to the training data as a new ROI image.

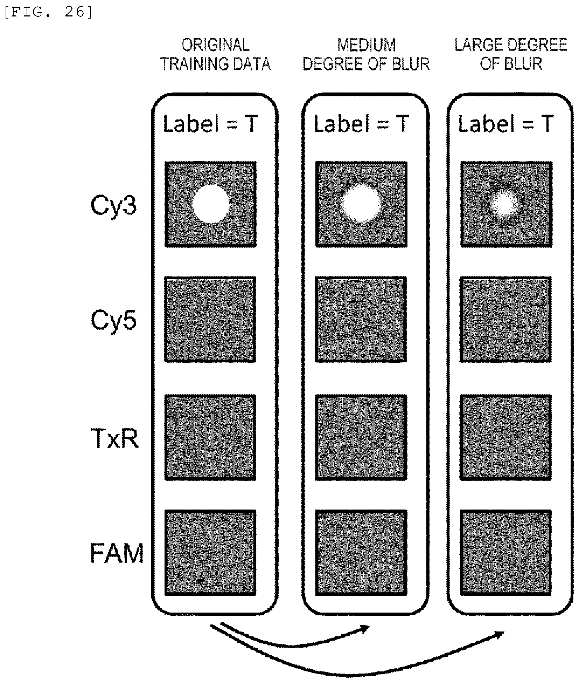

As an example in FIG. 26, images that are appropriately blurred by applying filter processing to the ROI image in the original training data are generated, and these images are added to the training data to train the predictor parameters. By such processing, robustness of the base prediction against focus deviation can be improved.

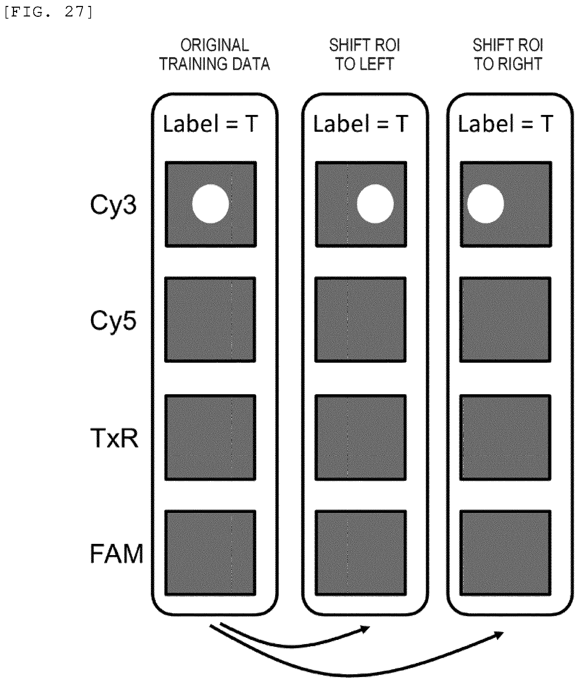

As another example in FIG. 27, images obtained by performing shift processing on the ROI image in the original training data are generated, and these images are added to the training data to train the predictor parameters. By such processing, the robustness of the base prediction against variations in the registration accuracy can be improved.

In addition to the above examples, an image to which processing such as rotation, enlargement, and reduction is applied may be added.

As described above, in the sixth embodiment, the parameters of the base predictor are trained by adding the ROI images subjected to various image processing to the training data, so that the robustness of the base predictor can be improved.

[Seventh Embodiment]

In a seventh embodiment, the correct information to be added to the training data is screened in the training data update step (S193) in the training processing (FIG. 19) of the learning unit 804 described in the fourth embodiment. In the fourth embodiment, as described above with reference to FIG. 22, the correct information (2100 to 2104) is included in the training data for all the bases aligned in step S192. However, in the case of the aligned bases, there is a possibility that an undesired base is included in the training data. In the present embodiment, the undesired base is determined and excluded from the training data by the screening processing, and then the training data is updated. Hereinafter, an example of determining a base to be excluded from the training data will be described.

(1) Separation of Colony

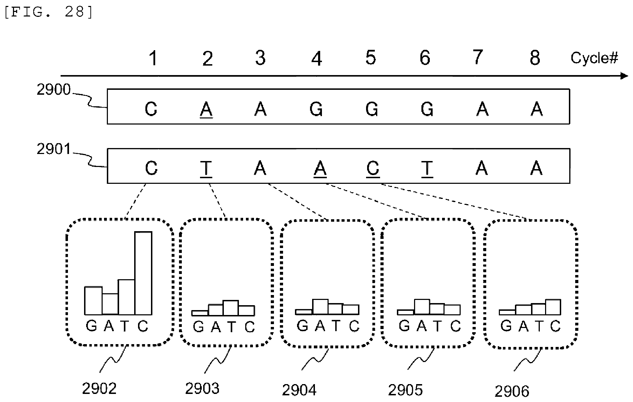

FIG. 28 shows a concept of detecting separation of the colony from the flow chip. This figure shows an example in which a base sequence 2901 called in a certain colony is aligned with a reference sequence 2900. In this example, mismatching with the reference sequence occurs in the second cycle, the fourth cycle, the fifth cycle, and the sixth cycle. In FIG. 28, 2902 to 2906 show signal intensities of four fluorescent images (G, A, T, and C correspond to fluorescent substances FAM, Cy5, Cy3, and TxR, respectively) at colony center positions, which correspond to the base sequence 2901. The signal intensity may be directly obtained from the fluorescent image, or may be obtained through a calculation process such as linear transformation using a color transformation matrix measured in advance.

In 2902, a signal intensity corresponding to C is high, and is also more remarkable than signal intensities of other bases. In contrast, all the signal intensities are low in 2903 and thereafter. In such a case where the fluorescence intensity is low as a whole in a certain cycle and thereafter, there is a possibility that the colony is separated from the flow chip and fluorescence cannot be obtained in the chemical treatment of each cycle described with reference to FIG. 2. When such separation of the colony is considered because the fluorescence intensities of all the fluorescent substances are lower than, for example, a threshold value, the colony is excluded from the training data regardless of matching or mismatching with the reference sequence.

(2) Base Mutation

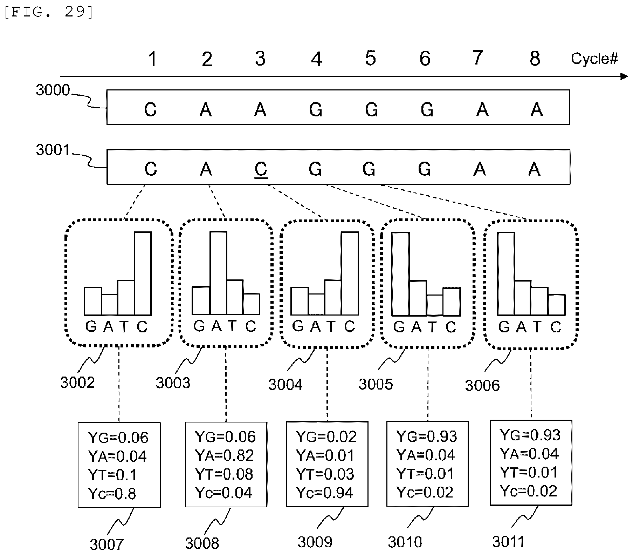

FIG. 29 is a conceptual diagram for detecting base mutations. This figure shows an example in which a base sequence 3001 called in a certain colony is aligned with a reference sequence 3000. In this example, mismatching with the reference sequence occurs in the third cycle. In this figure, 3002 to 3006 show signal intensities of four fluorescent images at the respective colony center positions. In 3002 to 3006 in this figure, a signal intensity corresponding to the base sequence 3001 called in each of the colonies is more remarkable than those of the other bases, and the signal intensity is also higher than that in FIG. 28.

In particular, in 3004 in FIG. 29 in which mismatching occurs in the third cycle, the called base "C" is also more remarkable than other bases. In this way, it is presumed that the reliability of the base that is called with a remarkable signal intensity is relatively high. Therefore, when a signal intensity of the called base is more remarkable than those of other bases in a cycle in which the mismatching has occurred and several cycles previous and next to the cycle, it is presumed that the base is different from the base of the reference sequence 3000 because a mutation has occurred in the cycle in which the mismatching has occurred. That is, the information of the reference sequence 3000 is unreliable when such a mutation occurs. Therefore, the base whose mutation is detected in this way is excluded from the training data.

As an example of an index indicating how much the signal intensity of the called base is more remarkable than other bases as described above, the following expression may be used.

[Formula 5]

$$D = I\_call/\sum I \qquad \cdots (5)$$

Here, I_call represents the signal intensity of the called base, and the denominator is a sum of fluorescence intensities I of the four colors. By using such an index D, it may be determined whether the mismatched base is mutated.

As another example, there is a method of using the information of the likelihood output by the base prediction unit. In the base prediction processing (S137) in the base prediction unit 803 described in the first embodiment, the Softmax unit in the CNN described in FIG. 16 finally outputs the likelihood Yk of each base.

In 3007 to 3011 of FIG. 29, an example of the likelihood of each base in each cycle is shown. It can be said that the higher the likelihood of the base is, the higher the reliability of base identification is. Therefore, when the likelihood of the called base is high in a cycle in which the mismatching has occurred and several cycles previous and next to the cycle, it may be estimated that the base is different from the base of the reference sequence 3000 because a mutation has occurred in the cycle in which the mismatch has occurred. The base whose mutation is detected in this way is excluded from the training data.

As described above, in the seventh embodiment, when the training data is updated, the reliability of the base calling results is calculated based on the information such as the signal intensity and the likelihood of the fluorescent image of the aligned base, and it is determined whether to add the result as the training data based on the reliability. Accordingly, the quality of training data during training is improved, and the prediction accuracy of the base predictor can be improved.


[Eighth Embodiment]

In the fourth embodiment, the configuration in which the base prediction unit 803 and the learning unit are provided in the nucleic acid analyzer 100 has been described. In an eighth embodiment, an example of a system configuration in which a nucleic acid analyzer, a base prediction unit, and a learning unit are separated is shown.

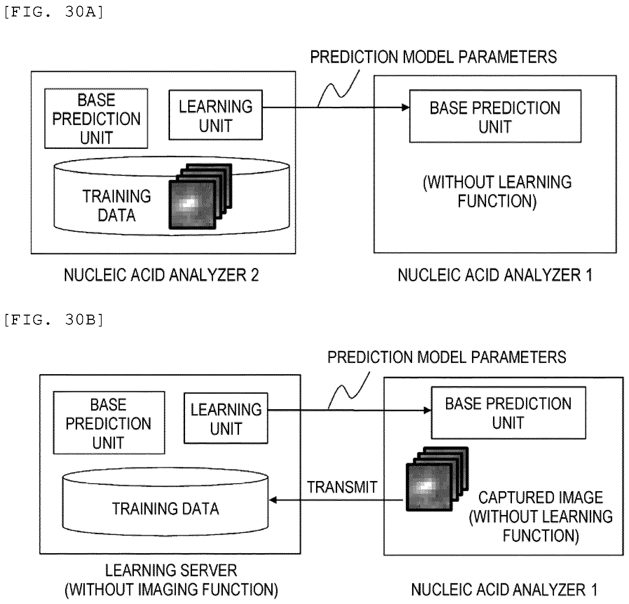

FIGS. 30A to 30C show a plurality of system configuration examples.

FIG. 30A shows a configuration of a system in which nucleic acid analyzers 1 and 2 include the same base prediction unit, the nucleic acid analyzer 2 performs training of the base prediction unit and transmits prediction model parameters obtained by the training to the nucleic acid analyzer 1. As training data used during the training, an image captured by the nucleic acid analyzer 2 is used. A typical operation example is a mode in which a measurement was performed using the nucleic acid analyzer 2 owned by a nucleic acid analyzer vendor to generate prediction model parameters, and the prediction model parameters are downloaded to the nucleic acid analyzer 1 owned by a user. Such a configuration example can be applied to a case where the variation between the apparatus is small. As an advantage of this configuration, the nucleic acid analyzer 1 owned by the user does not need to have a learning function, and thus the apparatus cost can be reduced.

FIG. 30B shows a configuration of a system in which the nucleic acid analyzer 1 and an external learning server include the same base prediction unit, and the learning server transmits prediction model parameters obtained by training of the base prediction unit using an image captured by the nucleic acid analyzer 1 to the nucleic acid analyzer 1. A typical operation example is a mode in which a nucleic acid analyzer vendor provides, as a learning server, a user with a computer having only a learning function, an image captured by the nucleic acid analyzer 1 owned by the user is transmitted to the learning server, and prediction model parameters obtained by learning performed on the server is downloaded to the nucleic acid analyzer 1. Such a configuration is effective when the variation between the devices is too large to be ignored and it is preferable to have prediction model parameters for each device. As in FIG. 30A, the nucleic acid analyzer 1 owned by the user does not need to have a learning function, and thus the apparatus cost can be reduced. However, a network capacity for transmitting an image to the server is required.

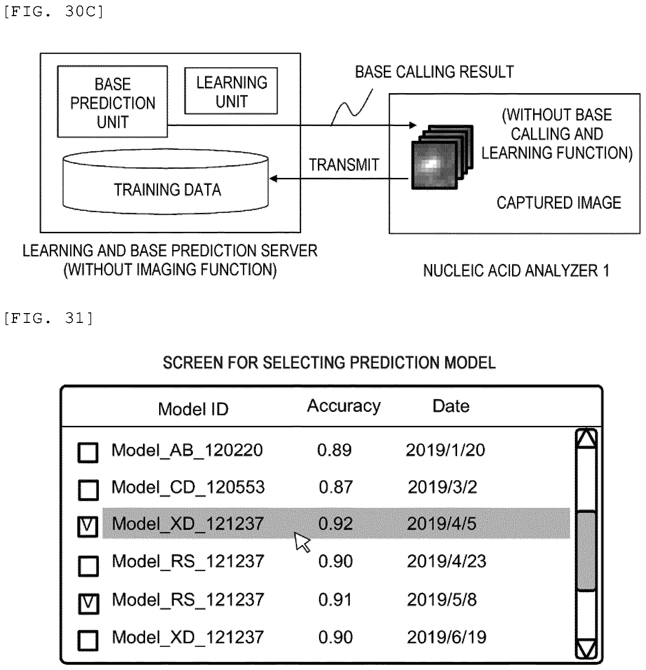

FIG. 30C is a configuration example in which the nucleic acid analyzer 1 is changed from the configuration of FIG. 30B and the base calling function is also transferred to an external server. In this configuration, as a function of the nucleic acid analyzer 1, only a colony image is captured, and all subsequent base callings are performed by the external server. The function is reduced as compared with FIG. 30B, and thus the apparatus cost can be reduced. However, a network capacity for transmitting an image to the server is required.

As described above, in the eighth embodiment, a system configuration in which the nucleic acid analyzer, the base prediction unit, and the learning unit are separated is adopted, so that the costs of the nucleic acid analyzer provided to the user, the base prediction processing function, and the learning processing function can be reduced.

[Ninth Embodiment]

In a ninth embodiment, several user interface examples in the embodiments described above will be described. These user interfaces are presented by the UI unit 808 in FIG. 6. The information may be presented by using a monitor screen of an external computer and a peripheral device such as a mouse and a keyboard.

(1) Selection in a Plurality of Predictors

FIG. 31 shows an example of a screen for performing selection in a plurality of base predictors in the configuration (FIG. 18) using the plurality of base predictors described in the third embodiment. In this figure, a list of a plurality of trained prediction model parameters existing in the nucleic acid analyzer is presented to the user, and a base predictor to be used for prediction is selected from the list. As an example in this figure, training accuracy of individual prediction parameters, creation date, and the like are presented. However, the invention is not necessarily limited thereto, and various information to be referred to in selecting the prediction parameter may be presented.

(2) Setting of Prediction Model for Each Cycle

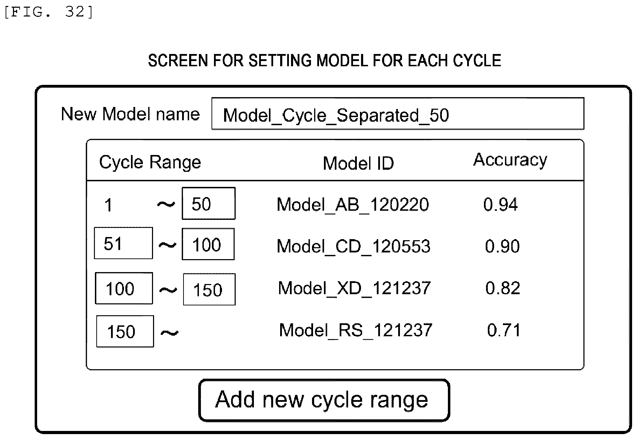

FIG. 32 shows an example of a setting screen for selectively using base predictors for each cycle in the configuration (FIG. 18) using the plurality of base predictors described in the third embodiment. In FIG. 32, different prediction models are set at intervals of 50 cycles, and a combination of the prediction models is defined as a new prediction model. As described in the second embodiment, as the cycle increases, the influence of fading between cycles accumulates. Therefore, the properties of the captured image change depending on the number of cycles. Therefore, there is a possibility that it is effective to switch the prediction model used for the base calling in accordance with the cycle. The example in which the models are switched every 50 cycles is shown in this figure, and the cycles may be further finely divided. For example, the models may be switched every cycle. In a model used in each cycle, it is desirable to use training data acquired in the cycle to be used.

(3) Selection of Data Set Used for Training of Base Prediction Model

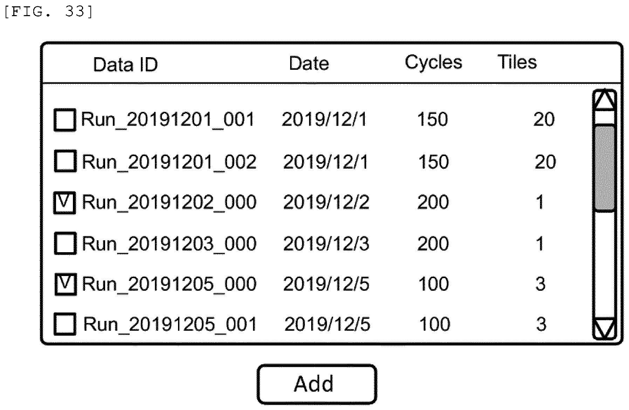

FIG. 33 shows an example of a setting screen for adding or deleting a data set used for training to or from a new or existing prediction model. The data set used for training may be image data stored in the storage unit 809 of a nucleic acid analyzer or image data stored in an external computer or a storage device.
FIG. 33 shows an example of a screen that presents, to a user, a list of data sets newly added to the training for a certain selected prediction model, and prompts the user to make a selection. By operating an "Add" button, a checked data set can be added.
On the same screen, a list of data sets that have already been used for training in the selected prediction model can be presented, and the user can be prompted to select image data to be excluded from those serving as training data during relearning. In this case, the checked data set is deleted by using a "Delete" button instead of the "Add" button. In this way, the image data set to be used for training may be changed based on the existing prediction model, and the image data set may be stored as a prediction model with a new name by a file name setting dialog (not shown).
In addition, the parameters for training described in the fourth and subsequent embodiments may be set for each piece of image data using a training setting screen (not shown). Some examples of such parameter setting items for training will be listed below. However, the invention is not necessarily limited thereto, and various parameters related to the training method of the base predictor described in the present embodiment may be set by the user.

(A) Applied Cycle Range

A range of a cycle of image data used for training is set. As described above, since the influence of fading varies depending on the cycle, it is effective to select which image is used in which cycle.

(B) Applied FOV

In the image data, which FOV image is to be used is set. This is because the properties of an image may change due to the influence of distortion or the like of the flow chip depending on a position of the FOV. In addition, it is also useful to limit the FOV in an application, for example, it is desired to use only a specific FOV for training instead of using all the FOVs.

(C) Size of Input ROI and the Number of Previous Cycles and Next Cycles

The setting may be changed for each prediction model in consideration of the degree of focus of an image or the influence of fading.

(D) CNN Network Configuration

Setting items of a known CNN, such as the number of network layers, the type of activation functions, the presence or absence of a Pooling layer, a learning rate, the number of epochs, and the number of mini-batches, may be changed for each prediction model.

(E) Selection of Additional Training or New Training

When a base prediction model is updated by adding the training data, it may be possible to set whether the prediction model is updated with the immediately preceding base prediction model as an initial value, or whether the prediction model is reset to newly recreate an initial value and relearning all the training data.

(F) Setting of Screening of Training Data

The conditions for screening during the update of the training data described in the seventh embodiment are set. The conditions include a threshold value of reliability, a threshold value of likelihood, a threshold value of signal intensities, and the like for determining a base not to be included in the training data.

Various nucleic acid reactions can be detected and nucleic acids such as DNA sequences can be analyzed using the nucleic acid analyzers or the base identification methods in the embodiments described above. The invention is not limited to the embodiments described above and includes various modifications. For example, the embodiments described above are described in detail for a better understanding of the invention, and the invention is not necessarily limited to embodiments including all configurations described above. The nucleic acid analyzers of the embodiments described above use a DNA fragment as a measurement and analysis target, and other biologically related substances such as RNA may be targeted in addition to DNA.

Further, although an example of creating a program that implements a part or all of the configurations, functions, and computers described above is mainly described, it is needless to say that a part or all of them may be implemented by hardware, for example, by designing an integrated circuit. That is, all or a part of the functions of the processing unit may be implemented by, for example, an integrated circuit such as an application specific integrated circuit (ASIC) or a field programmable gate array (FPGA) instead of the program.

Industrial Applicability

The invention can be used for nucleic acid analysis for measuring a biologically related substance.

Reference Signs List

| | |
|---|---|
| 100 | nucleic acid analyzer |
| 101, 102 | two-dimensional sensor |
| 102, 121 | imaging lens |
| 103 | band-pass filter |
| 104 | excitation filter |
| 105, | 120 dichroic mirror |
| 106 | filter cube |
| 107 | light source |
| 108 | objective lens |
| 109 | flow cell |
| 112, 115 | pipe |
| 113 | reagent container |
| 114 | reagent storage unit |
| 116 | waste liquid tank |
| 117 | stage |

| 118 | temperature control substrate |
| 119 | computer |
| 123 | analyzing area |
| 124 | field of view |
| 800 | base calling unit |
| 801 | registration unit |
| 802 | colony extraction unit |
| 803 | base prediction unit |
| 804 | learning unit |

**Claims**

1. A nucleic acid analyzer comprising:

   a base prediction unit configured to perform base prediction using, as an input, a plurality of images obtained by detecting luminescence from a biologically related substance disposed on a substrate;
   a registration unit configured to perform registration of the plurality of images relative to a reference image; and
   an extraction unit configured to extract a spot from the plurality of images, wherein
   the base prediction unit receives, as an input, an image including peripheral pixels around a position of the spot extracted from the plurality of images, extracts feature data of the image, and predicts a base based on the feature data.

2. The nucleic acid analyzer according to claim 1, wherein
   the plurality of images are obtained by detecting, by a sensor, a plurality of types of luminescence from a plurality of types of fluorescent substances incorporated into the biologically related substance, and the plurality of types of luminescence are different in at least one of the sensor for detection and an optical path to the sensor for detection.

3. The nucleic acid analyzer according to claim 1, wherein
   the base prediction unit is implemented by a predictor capable of performing supervised learning.

4. The nucleic acid analyzer according to claim 1, wherein
   the base prediction unit receives, in addition to an image in a cycle to be predicted, an image in at least one cycle selected from a previous cycle and a next cycle as an input.

5. The nucleic acid analyzer according to claim 1, wherein
   a plurality of the base prediction units are provided, and a base is predicted based on prediction results of the plurality of base prediction units.

6. A nucleic acid analysis method for performing base prediction by a base predictor receiving, as an input, a plurality of images obtained by detecting luminescence from a biologically related substance, the method comprising:

   executing a colony position determining stage and a base sequence determining stage, wherein
   in the colony position determining stage,

   registration processing of registering the plurality of images, and
   colony position determining processing of determining a colony position of the biologically related substance by extracting a spot from the plurality of images are executed, and

   in the base sequence determining stage,

   the base predictor receives, as an input, an image including peripheral pixels around the colony position extracted from the plurality of images, extracts feature data of the image, and predicts a base based on the feature data.

7. The nucleic acid analyzer according to claim 6, wherein
   the plurality of images are obtained by detecting, by a sensor, a plurality of types of luminescence from a plurality of types of fluorescent substances incorporated into the biologically related substance, and the plurality of types of luminescence are different in at least one of the sensor for detection and an optical path to the sensor for detection.

8. The nucleic acid analysis method according to claim 6, wherein

in the base sequence determining stage,
the base predictor receives, as the image including the peripheral pixels around the colony position extracted from the plurality of images, a set including a plurality of images captured at temporally different timings.

9. The nucleic acid analysis method according to claim 6, wherein
in the colony position determining processing, a position of the biologically related substance is determined by extracting a spot from the plurality of images captured at temporally different timings.

10. A machine learning method of a base predictor for performing base prediction using, as an input, a plurality of images obtained by detecting luminescence from a biologically related substance, the machine learning method comprising:

a first base prediction step of generating a first base prediction result based on the plurality of images;
a first training data generation step of generating first training data based on an alignment result between the first base prediction result and a reference sequence;
a predictor updating step of updating a parameter of the base predictor using the first training data generated in the first training data generation step;
a second base prediction step of generating a second base prediction result based on the plurality of images by using the base predictor updated in the predictor updating step;
a second training data generation step of generating second training data based on an alignment result between the second base prediction result and the reference sequence; and
a training data updating step of updating the first training data using the second training data.

11. The machine learning method according to claim 10, wherein
the base predictor receives, in addition to an image in a cycle to be subjected to base prediction, an image in at least one cycle selected from a previous cycle and a next cycle as an input.

12. The machine learning method according to claim 10, wherein

in at least one of the first training data generation step and the second training data generation step,
an image obtained by applying image processing to an image included in at least one of the first training data and the second training data is added to at least one of the first training data and the second training data.

13. The machine learning method according to claim 10, wherein

in at least one of the first training data generation step and the second training data generation step,
reliability of an image included in at least one of the first training data and the second training data is determined based on information of at least one of a signal intensity and likelihood, and an image to be used for at least one of the first training data and the second training data is selected based on the reliability.

14. The machine learning method according to claim 10, wherein
in the training data updating step, data in the second training data, which is not included in the first training data, is added to the first training data.

15. The machine learning method according to claim 10, further comprising:
a predictor reupdating step of updating a parameter of the base predictor using the first training data updated in the training data updating step.

[FIG. 1]

[FIG. 2]

| RUN | S21 |
|---|---|

S22

| CYCLE #1 | CYCLE #2 | - - - - - - - - - - - - - | CYCLE #M |
|---|---|---|---|

S23

| CHEMICAL TREATMENT | IMAGING PROCESSING | S24 |
|---|---|---|

S25

| IMAGING IN FOVI#1 | IMAGING IN FOV#2 | IMAGING IN FOV#3 | - - - - - - - | IMAGING IN FOV#N |
|---|---|---|---|---|

| S26 | S27 | S28 | S29 | S30 |
|---|---|---|---|---|
| STAGE MOVEMENT | FILTER (FAM/ Cy3) | EXPOSURE | FILTER (TxR/ Cy5) | EXPOSURE |

⬇         ⬇

IMAGE (FAM)     IMAGE (TxR)

IMAGE (Cy3)     IMAGE (Cy5)

[FIG. 3]

[FIG. 4]

(a)

(b) CY5

(c) CY3

(d) FAM

(e) TxR

[FIG. 5]

[FIG. 6]

[FIG. 7]

```
        ┌─────────────────────┐
        │     START BASE      │
        │      CALLING        │
        └─────────────────────┘
                  │
                  ▼
    ┌─────────────────────────┐
    │    COLONY POSITION      │      S90
    │   DETERMINING STAGE     │
    └─────────────────────────┘
                  │
                  ▼
    ┌─────────────────────────┐
    │    BASE SEQUENCE        │      S91
    │   DETERMINING STAGE     │
    └─────────────────────────┘
                  │
                  ▼
        ┌─────────────────────┐
        │  END BASE CALLING   │
        └─────────────────────┘
```

[FIG. 8]

S90

START COLONY POSITION
DETERMINING STAGE

Cycle=0 — S101

Cycle ← Cycle + 1
FOV=0 — S102

FOV ← FOV + 1 — S103

REGISTRATION PROCESSING — S104

SPOT EXTRACTION — S105

FINAL FOV? — S106
N
Y

NUMBER OF
COLONY DETERMINATION
CYCLES? — S107
N
Y

INTEGRATE COLONIES — S108

END COLONY POSITION
DETERMINING STAGE

[FIG. 9]

(a) cycle=N  (b) cycle=N+1

[FIG. 10]

S_1
O

n = 1

S_1    S_2    S_3    S_4
O      O      O      O

S_5    S_6    S_7    S_8
O      O      O      O

S_9    S_10   S_11   S_12
O      O      O      O

S_13   S_14   S_15   S_16
O      O      O      O

n = 16

(a) CALCULATION POSITION OF
POSITIONAL DEVIATION AMOUNT

O  O  O  O  O  O  O  O
O  O  O  O  O  O  O  O
O  O  O  O  O  O  O  O          ─── ANY PIXEL
O  O  O  O  O  O  O  O
O  O  O  O  O  O  O  O
O  O  O  O  O  O  O  O
O  O  O  O  O  O  O  O
O  O  O  O  O  O  O  O

(b) POSITIONS OF ANY PIXEL

[FIG. 11]

[FIG. 12]

COORDINATE SYSTEM OF REFERENCE IMAGE

[FIG. 13]

S91

START BASE SEQUENCE
DETERMINING STAGE

Cycle=0 — S131

Cycle ← Cycle + 1
FOV=0 — S132

FOV ← FOV + 1 — S133

REGISTRATION PROCESSING — S134

COLONY POSITION COORDINATE
TRANSFORMATION — S135

ROI IMAGE EXTRACTION — S136

BASE PREDICTION — S137

FINAL FOV? — S138

FINAL CYCLE? — S139

END BASE SEQUENCE
DETERMINING STAGE

[FIG. 14]

[FIG. 15]

[FIG. 16]

CNN

Conv ReLU Pooling Conv ReLU Pooling Affine ReLU | Affine Softmax

FEATURE DATA CALCULATOR

MULTINOMIAL CLASSIFIER

[FIG. 17]

ROI IN CYCLE (N-1)  ROI IN CYCLE (N)  ROI IN CYCLE (N+1)

FAM TxR  FAM TxR  FAM TxR
Cy3 Cy5  Cy3 Cy5  Cy3 Cy5

BASE PREDICTOR

A
G
C
T

[FIG. 18]

[FIG. 19]

```
         ┌─────────────────────┐
         │   START TRAINING    │
         └──────────┬──────────┘
                    │
                    ▼
         ┌─────────────────────┐
         │ INITIAL BASE        │ ～ S191
         │ PREDICTION          │
         └──────────┬──────────┘
                    │
                    ▼
         ┌─────────────────────┐
         │     ALIGNMENT       │ ～ S192
         └──────────┬──────────┘
                    │
         ┌──────────●
         │          │
         │          ▼
         │ ┌─────────────────────┐
         │ │ TRAINING DATA UPDATE│ ～ S193
         │ └──────────┬──────────┘
         │            │
         │            ▼
         │ ┌─────────────────────┐
         │ │ BASE PREDICTOR UPDATE│ ～ S194
         │ └──────────┬──────────┘
         │            │
         │            ▼
         │ ┌─────────────────────┐
         │ │   BASE PREDICTION   │ ～ S195
         │ └──────────┬──────────┘
         │            │
         │            ▼
         │ ┌─────────────────────┐
         │ │    REALIGNMENT      │ ～ S196
         │ └──────────┬──────────┘
         │            │
         │  Y         ▼
         └────────◇ UPDATE  ◇ ～ S197
                  ◇ CONTINUE?◇
                    └───┬───┘
                        │ N
                        ▼
         ┌─────────────────────┐
         │   DETERMINATION     │ ～ S198
         │  OF BASE PREDICTOR  │
         └──────────┬──────────┘
                    │
                    ▼
         ┌─────────────────────┐
         │    END TRAINING     │
         └─────────────────────┘
```

[FIG. 20]

[FIG. 21]

INITIAL PREDICTION RESULT

ALIGNED
(CORRECT)

ALIGNED
(INCORRECT)

2301

2300

[FIG. 22]

[FIG. 23]

CYCLE

1 2 3 4 5 6 ------------------

COLONY ID

1
2
3
4

(A) LINK DESTINATION OF IMAGE DATA
(B) POSITION
(C) PREDICTED BASE
(D) WHETHER BEING ALIGNED
(E) CORRECT BASE
(F) LIKELIHOOD OF EACH BASE
(G) WHETHER CORRECT INFORMATION IS
     INCLUDED IN TRAINING DATA

[FIG. 24]

INITIAL PREDICTION

SECOND PREDICTION

THIRD PREDICTION

FOURTH PREDICTION

[FIG. 25]

[FIG. 26]

[FIG. 27]

[FIG. 28]

[FIG. 29]

[FIG. 30A]

PREDICTION MODEL PARAMETERS

BASE PREDICTION UNIT

LEARNING UNIT

TRAINING DATA

NUCLEIC ACID ANALYZER 2

BASE PREDICTION UNIT

(WITHOUT LEARNING FUNCTION)

NUCLEIC ACID ANALYZER 1

[FIG. 30B]

PREDICTION MODEL PARAMETERS

BASE PREDICTION UNIT

LEARNING UNIT

TRAINING DATA

TRANSMIT

BASE PREDICTION UNIT

CAPTURED IMAGE (WITHOUT LEARNING FUNCTION)

LEARNING SERVER (WITHOUT IMAGING FUNCTION)

NUCLEIC ACID ANALYZER 1

[FIG. 30C]

BASE PREDICTION UNIT | LEARNING UNIT

BASE CALLING RESULT

TRAINING DATA

TRANSMIT

(WITHOUT BASE CALLING AND LEARNING FUNCTION)

CAPTURED IMAGE

LEARNING AND BASE PREDICTION SERVER
(WITHOUT IMAGING FUNCTION)

NUCLEIC ACID ANALYZER 1

[FIG. 31]

SCREEN FOR SELECTING PREDICTION MODEL

| Model ID | Accuracy | Date |
|---|---|---|
| ☐ Model_AB_120220 | 0.89 | 2019/1/20 |
| ☐ Model_CD_120553 | 0.87 | 2019/3/2 |
| ☑ Model_XD_121237 | 0.92 | 2019/4/5 |
| ☐ Model_RS_121237 | 0.90 | 2019/4/23 |
| ☑ Model_RS_121237 | 0.91 | 2019/5/8 |
| ☐ Model_XD_121237 | 0.90 | 2019/6/19 |

[FIG. 32]

SCREEN FOR SETTING MODEL FOR EACH CYCLE

New Model name | Model_Cycle_Separated_50

| Cycle Range | | Model ID | Accuracy |
|---|---|---|---|
| 1 ~ 50 | | Model_AB_120220 | 0.94 |
| 51 ~ 100 | | Model_CD_120553 | 0.90 |
| 100 ~ 150 | | Model_XD_121237 | 0.82 |
| 150 ~ | | Model_RS_121237 | 0.71 |

Add new cycle range

[FIG. 33]

| Data ID | Date | Cycles | Tiles |
|---|---|---|---|
| ☐ Run_20191201_001 | 2019/12/1 | 150 | 20 |
| ☐ Run_20191201_002 | 2019/12/1 | 150 | 20 |
| ☑ Run_20191202_000 | 2019/12/2 | 200 | 1 |
| ☐ Run_20191203_000 | 2019/12/3 | 200 | 1 |
| ☑ Run_20191205_000 | 2019/12/5 | 100 | 3 |
| ☐ Run_20191205_001 | 2019/12/5 | 100 | 3 |

Add

**INTERNATIONAL SEARCH REPORT**

International application No.

PCT/JP2020/018902

**A. CLASSIFICATION OF SUBJECT MATTER**
Int.Cl. C12M1/00(2006.01)i, G06T7/55(2017.01)i, C12Q1/6874(2018.01)i,
G16B40/20(2019.01)i
FI: C12M1/00A, C12Q1/6874Z, G06T7/55, G16B40/20
According to International Patent Classification (IPC) or to both national classification and IPC

**B. FIELDS SEARCHED**

Minimum documentation searched (classification system followed by classification symbols)
Int.Cl. C12M1/00, G06T7/55, C12Q1/6874 G16B40/20

Documentation searched other than minimum documentation to the extent that such documents are included in the fields searched
| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN)

**C. DOCUMENTS CONSIDERED TO BE RELEVANT**

| Category* | Citation of document, with indication, where appropriate, of the relevant passages | Relevant to claim No. |
|---|---|---|
| Y<br>A | WO 2017/203679 A1 (HITACHI HIGH-TECHNOLOGIES CORP.) 30 November 2017 (2017-11-30), particularly, paragraphs [0017]-[0087], [0135]-[0138], fig. 1, 5, 8 | 1-5<br>6-15 |
| Y<br>X<br>A | US 2019/0237160 A1 (QUANTUM-SI INCORPORATED) 01 August 2019 (2019-08-01), particularly, fig. 1-4, 39-41, paragraphs [0082], [0097]-[0104], [0172], [0200]-[0209] | 1-5<br>10-15<br>6-9 |
| X<br>A | JP 2018-180635 A (KONICA MINOLTA, INC.) 15 November 2018 (2018-11-15), particularly, claim 12, paragraphs [0033], [0039], fig. 8 | 6-9<br>1-5, 10-15 |
| A | US 2019/0213473 A1 (ILLUMINA, INC.) 11 July 2019 (2019-07-11), entire text | 1-15 |
| A | JP 2012-065081 A (HITACHI, LTD.) 29 March 2012 (2012-03-29), entire text | 1-15 |

☐ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 June 2020 | 14 July 2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| International application No. |
| --- |
| PCT/JP2020/018902 |

**Box No. II    Observations where certain claims were found unsearchable (Continuation of item 2 of first sheet)**

This international search report has not been established in respect of certain claims under Article 17 (2)(a) for the following reasons:

1. ☐ Claims Nos.:
    because they relate to subject matter not required to be searched by this Authority, namely:

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

```
Document 1: JP 2020-000060 A (HITACHI HIGH-TECHNOLOGIES CORP.) 09 January
2020, particularly, claims 1-17, paragraphs [0020]-[0057]
& WO 2020/003823 A1
```

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**       ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet) (January 2015)

**INTERNATIONAL SEARCH REPORT**

International application No.

PCT/JP2020/018902

**Box No. III    Observations where unity of invention is lacking (Continuation of item 3 of first sheet)**

This International Searching Authority found multiple inventions in this international application, as follows:

Claims 1-5 have the special technical feature of "a nucleic acid analysis device provided with: a base prediction part for performing a base prediction using, as an input, multiple images in which light emitted from a biologically related substance disposed on a substrate is detected; a positioning part that positions the multiple images at a reference image; and an extraction part that extracts a bright spot from the multiple images, wherein the base prediction part uses, as an input, an image within the multiple images that includes neighboring pixels of the position of the extracted bright spot, extracts a feature value of the image, and predicts a base on the basis of the feature value"; thus these claims are classified as invention 1.

Next, claims 6-9 are now examined. Claim 6 does not include the definition wherein the biologically related substance is disposed on a substrate, and the description does not define what the colony recited in claim 6 is like. Then, claims 6-9 have the common technical feature between these claims and claim 1 classified as invention 1 of "performing a base prediction using, as an input, multiple images in which light emitted from a biologically related substance is detected, by a positioning process for positioning the multiple images, and steps including extracting a feature value of the multiple images and predicting a base on the basis of the feature value". However, this technical feature does not make a contribution over the prior art in light of the disclosure of document 1. Apart from this feature, there are not the same or corresponding special technical features between claims 6-9 and claim 1. Furthermore, claims 6-9 do not depend from claim 1, and are not substantially identical to or similarly closely related to any of the claims classified as invention 1. Accordingly claims 6-9 are classified as invention 2.

Claims 10-15 have the common technical feature between these claims, and claim 1 classified as invention 1 and claim 6 classified as invention 2 of "performing a base prediction using, as an input, multiple images in which light emitted from a biologically related substance is detected". However, this technical feature does not make a contribution over the prior art in light of the disclosure of document 1. Apart from this feature, there are not the same or corresponding special technical features between claims 10-15 and claim 1 or 6. Accordingly claims 10-15 cannot be identified as either of inventions 1 and 2, and claims 10-15 are classified as invention 3.

Form PCT/ISA/210 (continuation of first sheet) (January 2015)

INTERNATIONAL SEARCH REPORT
Information on patent family members

International application No.

PCT/JP2020/018902

```
WO 2017/203679 A1  30 November 2017    (Family: none)

US 2019/0237160 A1 01 August  2019     WO 2019/147904 A1
                                       particularly, fig. 1-4, 39-41,
                                       paragraphs [0082], [0097]-[0104],
                                       [0172], [0200]-[0209]
                                       TW 201935294 A

JP 2018-180635 A   15 November 2018    US 2018/0286040 A1
                                       particularly, claim 12,
                                       paragraphs [0062], [0070], fig. 8

US 2019/0213473 A1 11 July 2019        WO 2019/136284 A1
                                       entire text

JP 2012-065081 A   29 March 2012       (Family: none)
```

Form PCT/ISA/210 (patent family annex) (January 2015)

54

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020000060 A
- WO 2017203679 A